# EUROPEAN PATENT APPLICATION

(11) **EP 4 498 778 A1**
(43) Date of publication of application: **29.01.2025**
(21) Application number: 23774557.5
(22) Date of filing: 09.03.2023
(51) Int. Cl.: H10K 50/11, C09K 11/06, G06N 20/00, H10K 71/00, H10K 85/00

(54) **LIGHT-EMITTING ELEMENT AND PRODUCTION METHOD THEREFOR, LIGHT-EMITTING COMPOUND AND PRODUCTION METHOD THEREFOR, COMPOSITION AND PRODUCTION METHOD THEREFOR, INFORMATION PROCESSING METHOD, INFORMATION PROCESSING DEVICE, PROGRAM, METHOD FOR PROVIDING LIGHT-EMITTING COMPOUND, AND DATA GENERATION METHOD**

(30) Priority: 22.03.2022 JP 2022045845
(71) Applicant: Sumitomo Chemical Company, Limited, Tokyo 103-6020 (JP)
(72) Inventor: BANDO, Akinori, Ichihara-shi, Chiba 299-0195 (JP); SAITO, Takakazu, Tsukuba-shi, Ibaraki 300-3294 (JP); ISHITOBI, Masamitsu, Tsukuba-shi, Ibaraki 300-3294 (JP); NISHIDA, Toshihiko, Tsukuba-shi, Ibaraki 300-3294 (JP); YOSHIOKA, Ken, Osaka-shi, Osaka 554-8558 (JP)
(74) Representative: Hoffmann Eitle
(86) International application number: PCT/JP2023/009007
(87) International publication number: WO 2023/181958

(57) **Abstract**

Provided are an information processing method and the like in which it is possible to efficiently obtain a compound capable of having a good property.

In the information processing method, includes acquiring a spectral indicator obtained by quantum chemical calculation in a plurality of compounds to be candidates, classifying,on the basis of the acquired spectral indicator, each of the compounds into a group in which the spectral indicator satisfies a predetermined condition and extracting a group in which the spectral indicator does not satisfy the predetermined condition, and a light emitting compound classified into the group in which the spectral indicator satisfies the predetermined condition.

## Description

### Technical Field

The present invention relates to a light emitting element and a method for producing the same, a light emitting compound and a method for producing the same, a composition and a method for producing the same, an information processing method, an information processing device, a program, a method for providing a light emitting compound, and a data creation method.

### Background Art

The commercialization of a light emitting element (organic EL element) using an organic compound or an organic metal complex as a light emitting substance has progressed. In the organic EL element, an organic compound layer containing a light emitting material is interposed between a pair of electrodes, and by applying a voltage, electrons and holes injected from the electrode are rebonded such that the light emitting substance is in an excited state, and when the excited state is returned to a ground state, light is emitted. In accordance with the light emitting substance used in the light emitting element, the light emitting property thereof is different, and thus, research and development on a light emitting substance having an excellent light emitting property has progressed.

Recently, in the research and development of a novel substance or an alternative substance, a materials informatics (MI) technology that efficiently performs material development by using an information processing technology such as statistical analysis has been proposed (for example, Patent Literature 1).

### Citation List

### Patent Literature:

Patent Literature 1: Japanese Patent Application Laid-Open Publication No. 2019-87107

### Summary

### Technical Problems

In order to search for a compound capable of satisfying a desired property by using the MI technology, it is necessary to apply MI to a massive number of candidate compounds and evaluate the property of each of the compounds. In order to extract the compound capable of satisfying the desired property, a lot of calculation cost and time are still required.

A main object of the present disclosure is to provide an information processing method and the like in which it is possible to efficiently obtain a compound capable of having an good property.

### Solution to Problems

A light emitting element according to one aspect of the present disclosure is a light emitting element including an anode, a cathode, and a light emitting layer provided between the anode and the cathode, in which the light emitting layer contains a light emitting compound satisfying SpDiam_A of 5.0 or more, AATSC2c of 0.003 or less, MATS5s of 0.17 or less, GATS6c of 0.6 or more, and AETA_beta of 1.6 or less in a molecular descriptor by mordred.

A light emitting compound according to one aspect of the present disclosure satisfies SpDiam_A of 5.0 or more, AATSC2c of 0.003 or less, MATS5s of 0.17 or less, GATS6c of 0.6 or more, and AETA_beta of 1.6 or less in a molecular descriptor by mordred.

A composition according to one aspect of the present disclosure contains the light emitting compound described above, and at least one type selected from the group consisting of a hole transport material, a hole injection material, an electron transport material, an electron injection material, a light emitting material, an antioxidant, and a solvent.

A method for producing a light emitting compound according to one aspect of the present disclosure includes a preparation step of preparing a plurality of compounds, and an extraction step of extracting a light emitting compound, from the plurality of compounds obtained in the preparation step, in which in the extraction step, the light emitting compound satisfying at least one of SpDiam_A of 5.0 or more, AATSC2c of 0.003 or less, MATS5s of 0.17 or less, GATS6c of 0.6 or more, AETA_beta of 1.6 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, and n6ARing of 0 or more and 10 or less in a molecular descriptor by mordred is extracted.

A method for producing a light emitting compound according to one aspect of the present disclosure includes a preparation step of preparing a plurality of compounds, and an extraction step of extracting a light emitting compound in which a half width of a light emitting spectrum is less than a predetermined value, from the plurality of compounds obtained in the preparation step, in which in the extraction step, a light emitting compound satisfying at least one of SpDiam_A of 5.0 or more, AATSC2c of 0.003 or less, MATS5s of 0.17 or less, GATS6c of 0.6 or more, AETA_beta of 1.6 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, and n6ARing of 0 or more and 10 or less in a molecular descriptor by mordred is extracted.

A method for producing a composition according to one aspect of the present disclosure includes a step of mixing the light emitting compound produced by the method described above with at least one type selected from the group consisting of a hole transport material, a hole injection material, an electron transport material, an electron injection material, a light emitting material, an antioxidant, and a solvent.

A method for producing a light emitting element according to one aspect of the present disclosure is a method for producing a light emitting element including an anode, a cathode, and a light emitting layer provided between the anode and the cathode, includes a step of forming the light emitting layer by using the light emitting compound produced by the method described above.

**In** an information processing method according to one aspect of the present disclosure, includes acquiring a spectral indicator obtained by quantum chemical calculation in a plurality of compounds to be candidates. On the basis of the acquired spectral indicator, each of the compounds is classified into a group in which the spectral indicator satisfies a predetermined condition and a group in which the spectral indicator does not satisfy the predetermined condition. A light emitting compound classified into the group in which the spectral indicator satisfies the predetermined condition is extracted.

A method for producing a light emitting compound according to one aspect of the present disclosure includes a step of extracting a light emitting compound by the information processing method described above, and a step of obtaining the extracted light emitting compound.

In an information processing method according to one aspect of the present disclosure includes generating a plurality of compounds to be candidates. A value for each molecular descriptor of each of the generated compounds is acquired. A spectral indicator by inputting the obtained value of the molecular descriptor of each of the compounds to a model trained to output a spectral indicator of a compound when a value of a molecular descriptor of the compound is input. A light emitting compound in which the specified spectral indicator satisfies a predetermined condition is extracted.

A method for producing a light emitting compound according to one aspect of the present disclosure includes a step of extracting a light emitting compound by the information processing method described above, and a step of obtaining the extracted light emitting compound.

In a method for providing a light emitting compound according to one aspect of the present disclosure includes outputting a light emitting compound extracted from a plurality of compounds to be candidates generated by a computer. The light emitting compound in which a spectral indicator specified using a model trained to output a spectral indicator of a compound when a value of a molecular descriptor of the compound is input, satisfies the predetermined condition.

In a data creation method according to one aspect of the present disclosure includes storing a plurality of compounds to be candidates generated by a computer, and a spectral indicator calculated by quantum chemical calculation with respect to each of the compounds in association with each other. A value for each molecular descriptor in each of the compounds is further stored in association with each of the compounds.

In an information processing method according to one aspect of the present disclosure includes acquiring a condition of a desired property for a compound. A plurality of compounds to be candidates are generated by a computer. A value for each molecular descriptor of each of the generated compounds is acquired. The compound in which the property of the compound satisfies the condition is extracted from each of the generated compounds by using a model trained to output a property of a compound when a value of a molecular descriptor of the compound is input. A property is calculated by quantum chemical calculation with respect to the extracted compound. The compound in which the property calculated by the quantum chemical calculation satisfies the condition is specified. The specified compound and the property of the compound are output.

In an information processing method according to one aspect of the present disclosure includesreceiving a condition of a desired property for a compound. A plurality of molecular descriptors of the compound and a range of a value of each of the molecular descriptors satisfying the received condition are acquired. The plurality of molecular descriptors and the range of the value of each of the molecular descriptors, which are acquired, are displayed.

### Advantageous Effects of Invention

According to the present disclosure, it is possible to efficiently obtain the compound capable of having a good property.

### Brief Description of Drawings

FIG. 1 is a block diagram illustrating a configuration example of an information processing device 1 according to an embodiment.
FIG. 2 is a drawing illustrating a content example of compound information stored in a compound DB.
FIG. 3 is an explanatory drawing illustrating an outline of a learning model.
FIG. 4 is a flowchart illustrating an example of a processing procedure relevant to primary extraction.
FIG. 5 is a flowchart illustrating an example of a processing procedure relevant to secondary extraction.
FIG. 6 is a drawing illustrating mapping data.
FIG. 7 is a drawing illustrating the mapping data.
FIG. 8 is a schematic view illustrating an example of a result screen displayed on a display unit.
FIG. 9 is a block diagram illustrating a configuration example of an information processing device and an information terminal device in a modification example.
FIG. 10 is a flowchart illustrating an example of a processing procedure executed by an information terminal device.
FIG. 11 is a drawing illustrating mapping data of a compound shown in Table 1 and Table 2.
FIG. 12 is a drawing illustrating the mapping data of the compound shown in Table 1 and Table 2.

### Description of Embodiments

The present disclosure will be described in detail with reference to the drawings illustrating the embodiment thereof. First, an information processing method using an information processing device according to an embodiment will be described.

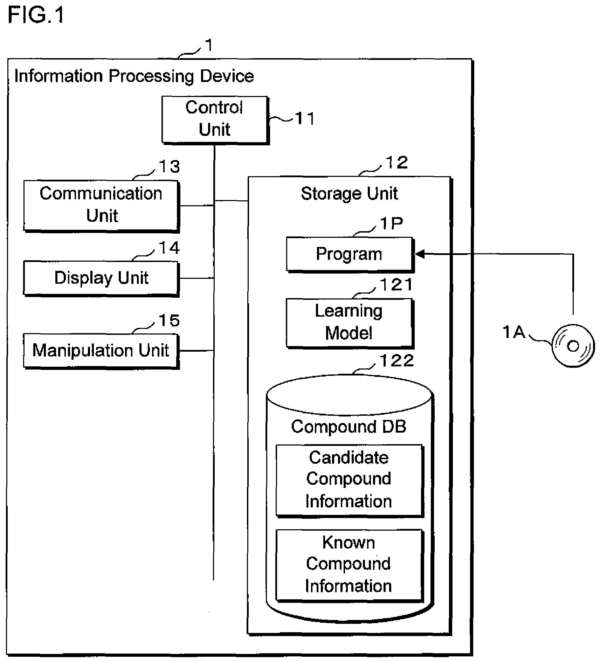

FIG. 1 is a block diagram illustrating a configuration example of an information processing device 1 according to an embodiment. The information processing device 1 is a device capable of processing various types of information and transmitting and receiving information, and for example, is a server computer, a personal computer, a quantum computer, and the like. The information processing device 1 functions as an extraction device extracting a compound capable of having a desired property or a novel compound capable of having a desired property, from compounds to be a plurality of candidates.

In this embodiment, processing contents implemented by the information processing device 1 will be described by using an example of applying the information processing device to the extraction of a light emitting compound capable of having a good light emitting spectrum. The good light emitting spectrum may be a light emitting spectrum at which the light emitting efficiency (the light extraction efficiency) of the light emitting compound can be improved. An application target of this embodiment is not limited to the light emitting compound, and for example, and this embodiment can be applied to the extraction of various compounds capable of having various properties, such as the extraction of a battery material capable of having a good charge and discharge performance.

In a light emitting element, a light emitting layer containing a light emitting compound that is a light emitting substance is interposed between a pair of electrodes, and by applying a voltage, the light emitting compound emits light. The spectrum of the light emitted from the light emitting compound is specific to the light emitting compound, and by using different types of light emitting compounds, it is possible to obtain the light emitting element emitting light with various colors and intensities.

The light emitting efficiency of the light emitting compound is affected by the light emitting of the light emitting compound. For example, by narrowing the spectrum width of the light emitting compound, it is possible to reduce a light emitting loss and further improve the light emitting efficiency. In this embodiment, as the light emitting compound that is capable of having a good light emitting spectrum and can be excellent in the light emitting efficiency, a light emitting compound in which the spectral indicator of the light emitting compound satisfies a predetermined condition is extracted.

The spectral indicator indicates a value relevant to the light emitting spectrum of the light emitting compound. Examples of the spectral indicator include a spectrum width, a spectrum intensity, an intensity ratio of a first peak and a second peak, the standard deviation of a spectrum shape, and the like. The spectrum width may be the half width of the light emitting spectrum. The half width may be a full width at half maximum (FWHM), or may be a half width at half maximum (HWHM). The spectrum width may be a width from a portion where the intensity of the second peak on a low energy side is 50% of the second peak to the first peak portion, in consideration of the second peak of the spectrum. In the following description, as the spectral indicator, the spectrum width (FWHM) is used.

The information processing device 1 includes a control unit 11, a storage unit 12, a communication unit 13, a display unit 14, a manipulation unit 15, and the like. The information processing device 1 may be a multicomputer consisting of a plurality of computers, or may be a virtual machine that is virtually constructed by software.

The control unit 11 is an arithmetic processing device including a central processing unit (CPU), a graphics processing unit (GPU), and the like. By using a built-in memory such as a read only memory (ROM) or a random access memory (RAM), the control unit 11 executes various computer programs stored in the ROM or the storage unit 12, and controls the operation of each hardware unit described above. The control unit 11 may have the functions of a timer measuring the elapsed time from when a measurement start instruction is applied to when a measurement end instruction is applied, a counter counting the number, a clock outputting date and time information, and the like.

The storage unit 12 includes a non-volatile storage device such as a hard disk or a solid state drive (SSD). In the storage unit 12, various computer programs and data are stored. The storage unit 12 may be composed of a plurality of storage devices, or may be an external storage device connected to the information processing device 1. In the computer program stored in the storage unit 12, a program 1P for causing a computer to execute processing relevant to the extraction of the light emitting compound is included.

In the storage unit 12, a learning model 121 and a compound database (DB) 122 are stored. The learning model 121 is a machine learning model generated by machine learning. It is assumed that the learning model 121 is used as a program module configuring a part of artificial intelligence software. The compound DB 122 is a database storing compound information relevant to a plurality of compounds.

The computer program (a computer program product) stored in the storage unit 12 may be provided by a non-transitory recording medium 1A in which the computer program is readably recorded. The recording medium 1A is a portable memory such as a CD-ROM, a USB memory, and a secure digital (SD) card. The control unit 11 reads a desired computer program from the recording medium 1A by using a reading device, which is not illustrated, and stores the read computer program in the storage unit 12. Alternatively, the computer program may be provided by communication. The program 1P may be composed of a single computer program or a plurality of computer programs, or may be executed on a single computer or executed on a plurality of computers connected to each other by a communication network.

The communication unit 13 includes a communication device for performing processing relevant to communication through a network such as the internet. The control unit 11 transmits and receives various types of information with respect to an external device through the communication unit 13.

The display unit 14 includes a display device such as a liquid crystal panel and an organic electro luminescence (EL) display. The display unit 14 displays various types of information, in accordance with an instruction from the control unit 11.

The manipulation unit 15 is an interface that receives the manipulation of a user, and for example, includes a keyboard, a touch panel device with a built-in display, a speaker, a microphone, and the like. The manipulation unit 15 receives the manipulation input from the user, and transmits a control signal according to manipulation contents to the control unit 11.

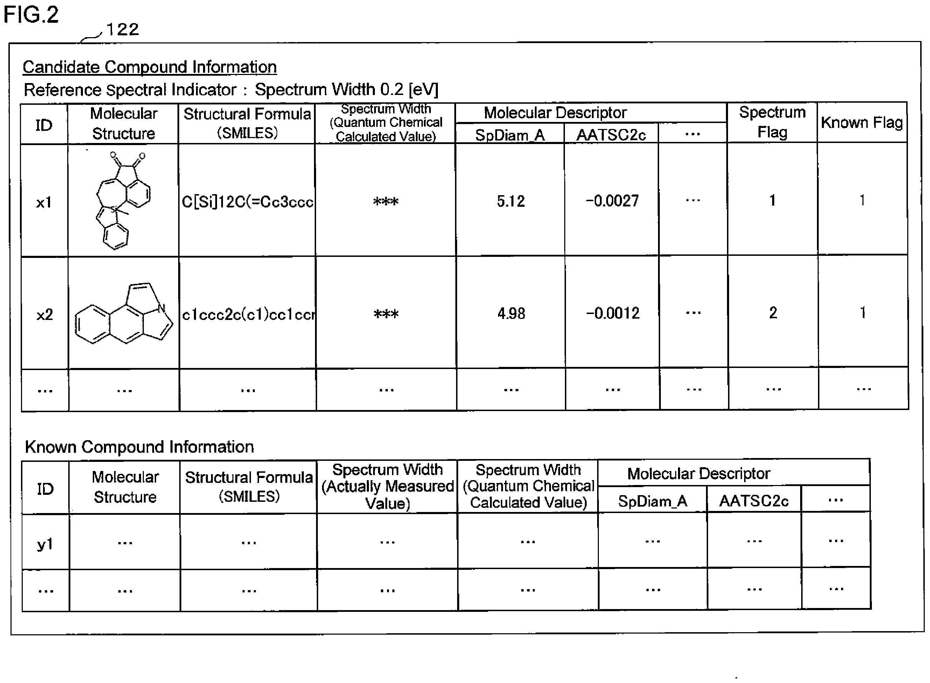

FIG. 2 is a drawing illustrating a content example of the compound information stored in the compound DB 122. In the compound DB 122, candidate compound information including compound information relevant to a compound to be a candidate (hereinafter, also simply referred to as a candidate compound), and known compound information including compound information relevant to a known compound are stored. The candidate compound indicates a compound that can be a new production candidate. The known compound is a compound with a known molecular structure, and indicates a compound that is not a novel compound.

In the candidate compound information, for example, a record is stored in which the molecular structure, the structural formula, the spectrum width (the quantum chemical calculated value), and the values of the molecular descriptor, the spectrum flag, the known flag, and the like of each of the compounds are associated with each other using an ID for identifying the compound as a key.

The structural formula is the chemical structural formula of the compound that is expressed in accordance with a simplified molecular input line entry system (SMILES) notation. Note that the notation of the chemical structural formula is not particularly limited insofar as it is possible to execute the quantum chemical calculation or the creation of the molecular descriptor described below with respect to the compound by stringifying the chemical structure.

The spectrum width (the quantum chemical calculated value) is the spectrum width of the compound that is obtained by the quantum chemical calculation. The quantum chemical calculation can be executed by using known quantum chemical calculation software, for example, Gaussian09 (manufactured by Gaussian, Inc.). The spectrum width is an example of the spectral indicator stored in the compound DB 122. In the compound DB 122, various other spectral indicators calculated by the quantum chemical calculation may be stored.

The molecular descriptor is quantified to make the structural characteristic, the physicochemical property, or the like of the compound easier to be handled by a calculator. The molecular descriptor can be calculated from the structural formula of the compound, and can be obtained by using known software, for example, mordred, RDKit, MOE, alvaDesc, PaDEL-Descriptor, Codessa, and the like. In this embodiment, molecular descriptor is calculated by mordred, the value of the molecular descriptor described below indicates a value calculated by mordred. In a molecular descriptor column, the values of a plurality of types of molecular descriptors defined by mordred are included.

In this embodiment, the molecular descriptor calculated by mordred as described above is used, but in accordance with software to be used, a descriptor with substantially the same contents, that is, a descriptor indicating the same or similar contents as those of the descriptor described above may be used.

The spectrum flag is a flag created in accordance with the spectral indicator of the candidate compound. Specifically, the spectrum flag is a flag indicating whether the spectral indicator of the candidate compound satisfies a predetermined condition. In a case where the spectral indicator is the spectrum width, the predetermined condition is whether the spectrum width is less than a threshold value. In the example illustrated in FIG. 2, in a case where the spectrum width is less than the threshold value, a flag "1" indicating that the spectral indicator satisfies the predetermined condition, and in a case where the spectrum width is the threshold value or more, a flag "2" indicating that the spectral indicator does not satisfy the predetermined condition are stored in a spectrum flag column.

The known flag is information indicating whether the candidate compound is known. In the example illustrated in FIG. 2, in a case where the candidate compound is known, a flag "1" indicating that the candidate compound is known is stored in the known flag column. In a case where the candidate compound is not known, the known flag column is a blank, or a flag indicating that the candidate compound is not known is stored in the known flag column.

In the known compound information, for example, the record is stored in which the molecular structure, the structural formula, the spectrum width (the actually measured value), the spectrum width (the quantum chemical calculated value), and the values of the molecular descriptor and the like of each of the compounds are associated with each other using the ID for identifying the compound as a key.

The spectrum width (the actually measured value) is the spectrum width of the compound that is obtained by an experiment. Other information contents stored in the known compound information are the same as the candidate compound information.

The information processing device 1, for example, collects the structural formula of the known compound by using a thesis, a specialized book, a known structural formula search means, and the like. The information processing device 1 may collect the compound by specializing in the light emitting compound having a light emitting property. The information processing device 1 acquires various actually measured values relevant to the collected compound, and various types of data obtained by calculation such as the quantum chemical calculation and the molecular descriptor, and accumulates the obtained value and data in the compound DB 122 as the known compound information. The compound information stored in the compound DB 122 is not limited to the example described above.

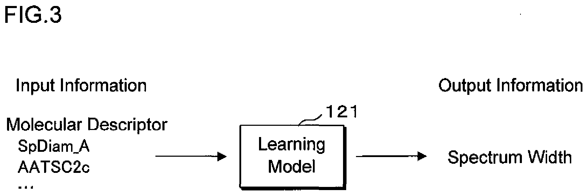

FIG. 3 is an explanatory drawing illustrating the outline of learning model 121. The learning model 121 is a machine learning model outputting the spectrum width of the compound using the values of a plurality of molecular descriptors in the compound as input. Note that the output value of the learning model 121 may be a spectral indicator other than the spectrum width.

The information processing device 1 generates in advance the learning model 121 by performing machine learning for learning predetermined training data. Then, the information processing device 1 estimates a spectrum width with respect to the molecular descriptor of a newly generated light emitting compound by using the learning model 121.

The learning model 121, for example, is random forest. The random forest is a classifier for classifying target data into target attribute matching the amount of characteristic by a plurality of decision trees, and obtains the final classification result by majority decision with respect to the output result obtained by each of the decision trees. In the case of handling regression problems, the representative value (for example the average value) of the output result obtained by each of the decision trees may be output as the final predicted value.

The learning model 121 is composed of a decision tree including a plurality of branch nodes and leaf nodes at the terminals of the branch nodes. In each of the decision trees, input data is classified in accordance with a condition on the way from the uppermost root node to the branch, and in the case of reaching the leaf node at the terminal, a value applied to the leaf node at the terminal is output as a predicted value. The value of the spectrum width is associated with each of the leaf nodes.

An explanatory variable input to the learning model 121 is the value of the plurality of molecular descriptors in the compound. An object variable output from the learning model 121 is the value of the spectrum width. In the generation of each of the decision trees, explanatory variables different from each other are used, and a spectrum width with respect to the explanatory variable is output.

Note that in the case of using the plurality of molecular descriptors as the explanatory variable, from the viewpoint of selecting a descriptor with high explanatory power for the spectrum width, for example, a predetermined number of molecular descriptors selected by Ridge regression, LASSO regression, or the like may be used. Since there are 1000 or more types of molecular descriptors defined by mordred, it is possible to reduce a calculation cost by selectively using the predetermined number of molecular descriptors.

The learning model 121 can be generated by preparing training data in which the value of the plurality of molecular descriptors is associated with a label indicating the spectrum width, and performing machine learning on an untrained model using the training data.

As the spectrum width to be ground truth, for example, the spectrum width calculated by the quantum chemical calculation can be used. The quantum chemical calculation can be executed by using known quantum chemical calculation software. Such quantum chemical calculation is known to obtain a highly accurate value with a small error from the actually measured value. **In** the consideration of the present inventors, it is found that the error between the actually measured value and the calculated value by the quantum chemical calculation is a negligible value. By using such a measured value by a simulation as the spectrum width of the training data, it is not necessary to collect the actually measured value by the experiment, and it is possible to easily and efficiently create more training data.

The spectrum width to be the ground truth is not limited to the spectrum width by the quantum chemical calculation, and may be the actually measured value by the experiment, or may be both of the spectrum width by the quantum chemical calculation and the actually measured value.

The information processing device 1 generates the decision tree while sequentially searching for the branch outputting the most excellent value using a loss function from the uppermost root node to the lower node by applying the input data included in the training data. The information processing device 1 adjusts parameters, for example, by using a gradient descent method to optimize (minimize) the loss function in the learning model 121. The information processing device 1 completes the learning by the loss function satisfying a predetermined criterion. In a case where the learning is completed, the learning model 121 trained to be capable of suitably recognizing the spectrum width of the compound with respect to the value of the molecular descriptor in the compound is constructed.

In the above description, an example has been described in which the learning model 121 is the random forest, but the configuration of the learning model 121 is not limited insofar as the spectrum width with respect to the molecular descriptor can be identified. The learning model 121, for example, may be a neural network such as a transformer, a convolution neural network (CNN), a recurrent neural network (RNN), and a long short term memory (LSTM), and other learning algorithms such as a support vector machine, logistics regression, and XGBoost (extreme gradient boosting) may be used.

The learning model 121 is not limited insofar as the learning model is generated and learned by the information processing device 1. The learning model 121 may be transmitted to the information processing device 1 as a learned model by an external server, and may be stored in the storage unit 12. The learning model 121 may be generated by an external server, and may be learned by the information processing device 1.

Hereinafter, the details of extraction processing of the light emitting compound that is executed by the information processing device 1 will be described.

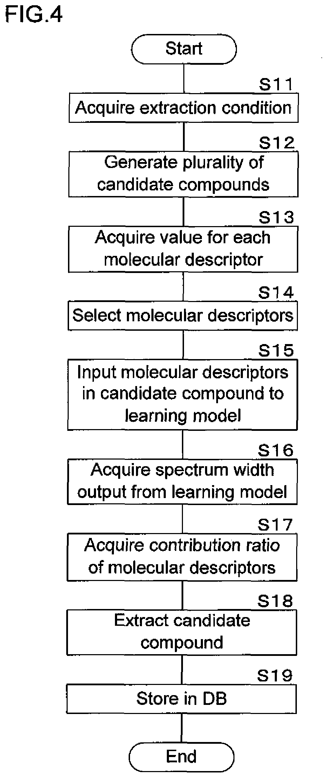

FIG. 4 is a flowchart illustrating an example of a processing procedure relevant to primary extraction. The processing in each of the following flowcharts may be executed by the control unit 11, in accordance with the program 1P stored in the storage unit 12 of the information processing device 1, may be attained by a dedicated hardware circuit (for example, FPGA or ASIC) provided in the control unit 11, or may be attained by a combination thereof.

The control unit 11 of the information processing device 1 acquires an extraction condition of the light emitting compound to be an extraction target (step S11). The extraction condition in this embodiment is the light emitting compound in which the spectrum width is less than the predetermined value, and the control unit 11 acquires the threshold value (the upper limit value) of the spectrum width of the light emitting compound. The control unit 11, for example, may acquire the extraction condition by receiving the input of the user obtained by manipulating the manipulation unit 15, or may acquire the extraction condition by receiving information transmitted from an external device connected through communication.

The control unit 11 generates a plurality of candidate compounds (step S12), and acquires a structural formula representing the molecular structure of each of the candidate compounds. The control unit 11 acquires a value for each molecular descriptor in each of the generated candidate compounds (step S13). Specifically, the control unit 11 calculates the value of the molecular descriptor from the structural formula of the candidate compound by using mordred.

The control unit 11, for example, selects the predetermined number of molecular descriptors used for the input to the learning model 121 from all of the molecular descriptors by using Ridge regression, LASSO regression, or the like (step S14). Note that the processing of step S14 may be omitted.

The control unit 11 inputs the value of the plurality of molecular descriptors in the selected candidate compound to the learning model 121 (step S15), and acquires the spectrum width output from the learning model 121 (step S16). The control unit 11 executes the processing described above with respect to each of the generated candidate compounds to obtain each spectrum width.

The control unit 11, for the plurality of selected molecular descriptors, calculates a contribution ratio to the prediction of the spectrum width in the learning model 121 (step S17). In a case where the learning model 121 is the random forest, the contribution ratio of the molecular descriptor corresponds to the degree of variable importance. The degree of importance, for example, can be calculated on the basis of out-of-bag (oob) data, a Gini coefficient, and the like. The contribution ratio of the molecular descriptor may be calculated by other methods such as Shapley additive explanation (SHAP) and local interpretable model-agnostic explanations (LIME). Note that the processing of step S17 may be omitted.

The control unit 11 extracts the candidate compound in which the spectrum width output from the learning model 121 is less than the threshold value, from all of the generated candidate compounds, on the basis of the acquired prediction result of the learning model 121 (step S18). The extraction of step S18 corresponds to the primary extraction. Note that as the threshold value in the primary extraction, a different value may be set independent from the threshold value acquired as the extraction condition. The primary extraction is not limited to using the threshold value, and the control unit 11, for example, may extract the predetermined number of candidate compounds in ascending order of the spectrum width output from the learning model 121.

The control unit 11, for each of the extracted candidate compounds, stores the molecular structure, the structural formula, the value for each of the molecular descriptors of the candidate compound in association with each other, in the candidate compound information of the compound DB 122 (step S19). The control unit 11 ends the primary extraction processing.

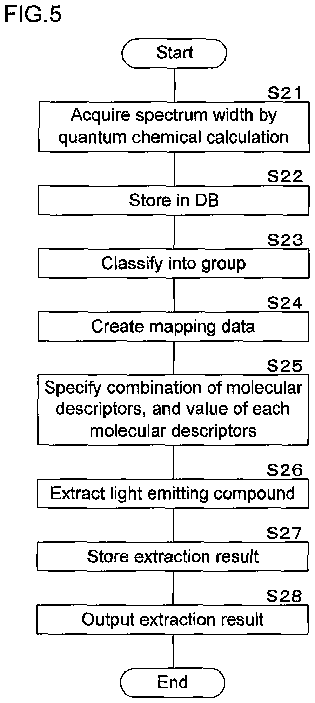

FIG. 5 is a flowchart illustrating an example of a processing procedure relevant to secondary extraction. The information processing device 1 may start the processing in FIG. 5, after the end of the primary extraction processing in FIG. 4.

The control unit 11 of the information processing device 1, for the plurality of candidate compounds extracted in step S18 of FIG. 4, acquires the spectrum width calculated by the quantum chemical calculation (step S21). The control unit 11 calculates the value of the spectrum width from the structural formula of the candidate compound by using predetermined quantum chemical calculation software.

The control unit 11 stores the acquired spectrum width and various flags created on the basis of the spectrum width of the candidate compound information of the compound DB 122 (step S22). Specifically, the control unit 11 determines whether the spectrum width is less than the threshold value, on the basis of the spectrum width by the quantum chemical calculation, and the threshold value of the spectrum width, and creates the spectrum flag according to the determination result. In addition, the control unit 11 determines whether the molecular structure of each of the generated candidate compounds is known, with reference to the known compound information, and creates the known flag with respect to the candidate compound of which the molecular structure is known.

The control unit 11 classifies each of the candidate compounds into any of a first group to a third group, on the basis of the spectrum width of each of the candidate compounds by the quantum chemical calculation, and the information stored in the compound DB 122 (step S23). Specifically, the control unit 11 groups each of the candidate compounds, on the basis of the type of spectrum flag associated with each of the candidate compounds and the presence or absence of the known flag. The candidate compound classified into the first group is a compound in which the spectrum width is less than the threshold value and the compound is not stored as the known compound. The candidate compound classified into the second group is a compound in which the spectrum width is less than the threshold value and the compound is stored as the known compound. The candidate compound classified into the third group is a compound in which the spectrum width is the threshold value or more.

The control unit 11 creates mapping data in which a combination value of the plurality of molecular descriptors is mapped for each of the groups (step S24). The control unit 11 specifies a combination of the molecular descriptors and the range of the value of each of the molecular descriptors that are capable of separating the first group from the second group and the third group, on the basis of the value for each of the molecular descriptors in each of the candidate compounds, and the group to which each of the candidate compounds belongs (step S25).

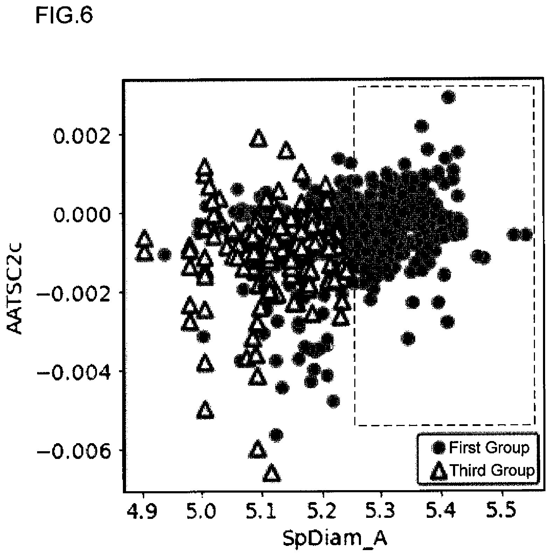

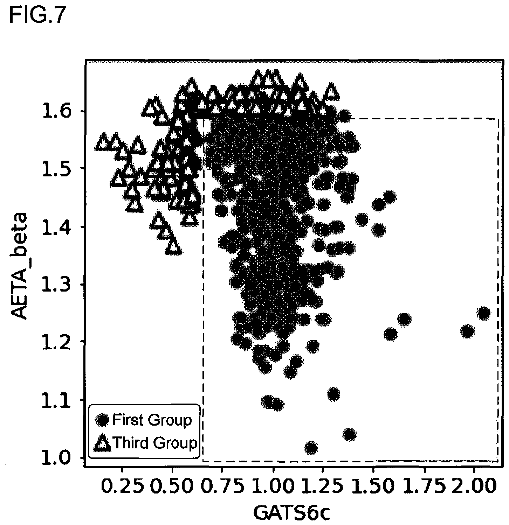

FIG. 6 and FIG. 7 are drawings illustrating the mapping data. As illustrated, the mapping data is a graph including different types of molecular descriptors on a vertical axis and a horizontal axis. On the graph, the combination value of the molecular descriptors in the plurality of candidate compounds is mapped.

In the example illustrated in FIG. 6, the vertical axis of the graph is AATSC2c, and the horizontal axis is SpDiam_A. The control unit 11, for each of the candidate compounds to be a mapping target, plots the values of AATSC2c and SpDiam_A stored in the candidate compound data on the graph. In FIG. 6, for the sake of easy explanation, the mapping data in which the compounds belonging to the first group and the third group are mapped is illustrated, but it is obvious that all of the compounds belonging to the first group to the third group may be mapped in the mapping data.

From the viewpoint of improving the accuracy of specifying the molecular descriptor described below, the control unit 11 may create the mapping data including the candidate compound other than the extraction target selected in the primary extraction, or may create the mapping data by including not only the candidate compound but also the known compound. For example, the control unit 11 may extract the known compound in which the spectrum width is less than the threshold value, with reference to the known compound information, and may map the extracted known compound in the mapping data as the compound belonging to the second group.

The mapping data is displayed such that the group belonging to each of the candidate compounds can be identified. The control unit 11, for example, changes the display mode of the marker of the value of the molecular descriptor, in accordance with the group, such as changing the form of the marker of the value of the molecular descriptor or a display color, in accordance with the group. Accordingly, it is possible for the user to efficiently grasp the plurality of pieces of information, and definitely recognize data for each of the groups.

The control unit 11 specifies the range of the value of the molecular descriptor capable of separating the candidate compound of the first group from the candidate compound of the other group by using the mapping data. The control unit 11 derives the upper limit value and the lower limit value of the molecular descriptor to include only the candidate compound of the first group, for each of the molecular descriptors, in accordance with the distribution status of the value of the molecular descriptor. The control unit 11 may specify only one of the upper limit value and the lower limit value. The control unit 11, for the combination of the molecular descriptors not capable of separating the first group, may determine that the combination is not appropriate, and may not specify the upper limit value and the lower limit value.

In the example illustrated in FIG. 6, the mapping is performed such that the candidate compound belonging to the first group is unevenly distributed on the right side of the graph. The control unit 11 specifies the upper limit value and the lower limit value of AATSC2c, and the upper limit value and the lower limit value of SpDiam_A, which are capable of cutting out a region indicated by a dashed line frame in FIG. 6.

The control unit 11 executes the same processing on the other combination of the molecular descriptors. Specifically, the control unit 11 creates the mapping data including new combinations of the molecular descriptors on the vertical axis and the horizontal axis, and specifies the range of the value for each of the molecular descriptors with respect to the combination of the molecular descriptors.

FIG. 7 illustrates an example of the mapping data according to the other combination of the molecular descriptors. FIG. 7 is the mapping data including AETA_beta on the vertical axis of the graph, and GATS6c on the horizontal axis. The control unit 11 further specifies the upper limit value and the lower limit value of AETA_beta, and the upper limit value and the lower limit value of GATS6c, which are capable of cutting out a region indicated by a dashed line frame at the right center in FIG. 7, on the basis of the mapping data illustrated in FIG. 7. Note that the dashed line frame illustrated in FIG. 6 and FIG. 7 simply indicates a concept of separating the first group, and does not limit the numerical value of the molecular descriptor.

The control unit 11 comprehensively evaluates a plurality of pieces of mapping data for each of different combinations of the molecular descriptors, and the range of the value of each of the molecular descriptors to specify the final combination of the molecular descriptors and range of the value of each of the molecular descriptors.

Specifically, the control unit 11, for the compound satisfying the range of the value of the molecular descriptor specified by first mapping data, creates second mapping data, and specifies a further range of the value of the molecular descriptor. Then, the compound satisfying the range of the value of the molecular descriptor specified by the first and second mapping data is specified by third mapping data. The control unit 11 finally acquires one combination of the molecular descriptors and the range of the value of each of the molecular descriptors that are capable of separating the first group from the other group. Note that the mapping data is not limited to indicating two types of molecular descriptors on two axes, but may include three or more types of molecular descriptors on the axes.

In the specifying processing of the combination of the molecular descriptors and the range, the control unit 11 may determine the degree of priority of the molecular descriptor used for the specifying processing, in accordance with the contribution ratio of each of the molecular descriptors. The control unit 11 preferentially uses the molecular descriptor in descending order of the contribution ratio, on the basis of the contribution ratio of each of the molecular descriptors calculated in step S17 of FIG. 4. The control unit 11 creates the combination in order from the molecular descriptor with a high contribution ratio, and specifies the range of the value of the molecular descriptor. In consideration of the contribution ratio of the molecular descriptor, it is possible to efficiently and effectively select the molecular descriptor highly affecting the separation of the candidate compound, from the plurality of molecular descriptors.

The control unit 11 may specify first the range of the value of the molecular descriptor, and then, may specify the combination of the molecular descriptors. The control unit 11, for example, extracts all of the known compounds in which the spectral indicator satisfies the predetermined condition, with reference to the compound DB 122, and acquires the value for each of the molecular descriptors in each of the extracted compounds. The control unit 11 specifies the maximum value and the minimum value in each of the acquired molecular descriptors, and sets the range of the specified maximum value and minimum value as the range of the value of the molecular descriptor. The control unit 11 specifies the combination of the molecular descriptors capable of separating the first group by using the molecular descriptor associated with the specified range.

Note that a method for specifying the combination of the molecular descriptors and the range of the value thereof is not limited to the above, and other known methods may be used.

The grouping of the candidate compound is not limited to the classification into the first group to the third group. For example, the candidate compound may be classified by whether the spectral indicator satisfies the predetermined condition, without considering whether the compound is the known compound. In this case, the candidate compound is classified into a group in which the spectral indicator satisfies the predetermined condition (a group in which the spectrum width is less than the threshold value) and a group in which the spectral indicator does not satisfy the predetermined condition (a group in which the spectrum width is the threshold value or more). The control unit 11 specifies the combination of the molecular descriptors and the range that are capable of separating the group in which the spectral indicator satisfies the predetermined condition from the group in which the spectral indicator does not satisfy the predetermined condition.

Returning to FIG. 5, the description will be continued. The control unit 11 extracts the candidate compound (the light emitting compound) satisfying the specified combination of the molecular descriptors and range of the value of each of the molecular descriptors, from the candidate compounds subjected to the primary extraction (step S26). By the processing of step S26, the light emitting compound belonging to the first group is extracted. The extraction of step S26 corresponds to the secondary extraction. In the secondary extraction, the control unit 11 may extract a predetermined number of light emitting compounds in ascending order of the spectrum width, among the light emitting compounds belonging to the first group.

The control unit 11 stores the secondary extraction result in the storage unit 12 (step S27). In a case where the light emitting compound classified into the first group by the secondary extraction is actually obtained by synthesis, the control unit 11 may create a new known flag in the candidate compound information as a new known compound. The control unit 11 adds the candidate compound information in which the known flag is newly created to the known compound information.

The control unit 11 outputs the obtained extraction result through the display unit 14 (step S28), and ends a series of processing.

Hereinafter, the processing contents in FIG. 4 and FIG. 5 will be described by using a specific numerical example.

In step S11, the control unit 11 receives a condition in which the spectrum width is less than 0.2 eV as the extraction condition. As the threshold value of the spectrum width, a value is set in which the spectrum width of the existing light emitting compound having a goodlight emitting spectrum is used as a criterion value, and a predetermined margin is added to the criterion value.

In step S12, the control unit 11, for example, generates a plurality of candidate compounds having a cyclohexane ring as a basic skeleton, and a different number of rings and different substituents.

In step S14, the control unit 11 acquires 50 types of molecular descriptors selected by Ridge regression, from a plurality of descriptors defined by mordred. Examples of the acquired molecular descriptor include SpDiam_A, AATSC2c, AATSC3s, MATS5s, GATS6c, GATS7i, C3SP2, AETA_beta, AETA_beta_s, SlogP_VSA5, n5aRing, n6Aring, nB, nARing, AXp-3d, SaasC, Vabc, and the like. The control unit 11, for each of the candidate compounds, calculates the value for each of 50 types of molecular descriptors. The control unit 11 predicts the spectrum width with the learning model 121 by using the calculated molecular descriptor, and calculates the contribution ratio of each of the molecular descriptors.

In step S25, the control unit 11 specifies the combination of the molecular descriptors and the range of the value of each of the molecular descriptors that are capable of separating a novel candidate compound group in which the spectrum width is less than 0.2 eV from a known compound group in which the spectrum width is less than 0.2 eV and a compound group in which the spectrum width is 0.2 eV or more.

As an example of the specifying result, for example, SpDiam_A of 5.0 or more, AATSC2c of 0.003 or less, MATS5s of 0.17 or less, GATS6c of 0.6 or more, and AETA_beta of 1.6 or less are obtained.

As the combination of the molecular descriptors and the range of the value of each of the molecular descriptors, SpDiam_A of 5.0 or more and 6.0 or less, AATSC2c of -0.010 or more and 0.003 or less, MATS5s of -0.30 or more and 0.17 or less, GATS6c of 0.6 or more and 3.0 or less, and AETA_beta of 0.5 or more and 1.6 or less can be further specified. When the combination of the molecular descriptors and the range of the value are within the above-described combinations and range, it is possible to more efficiently and effectively extract the light emitting compound capable of having a good light emitting spectrum by narrowing the spectrum width.

In addition, as the combination of the molecular descriptors and the range of the value of each of the molecular descriptors, nB of 0 or more and 2 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, n6ARing of 0 or more and 10 or less, nARing of 0 or more and 10 or less, AXp-3d of 0.10 or more and 0.20 or less, SaasC of 0 or more and 40 or less, and Vabc of 100 or more and 2000 or less can be further specified.

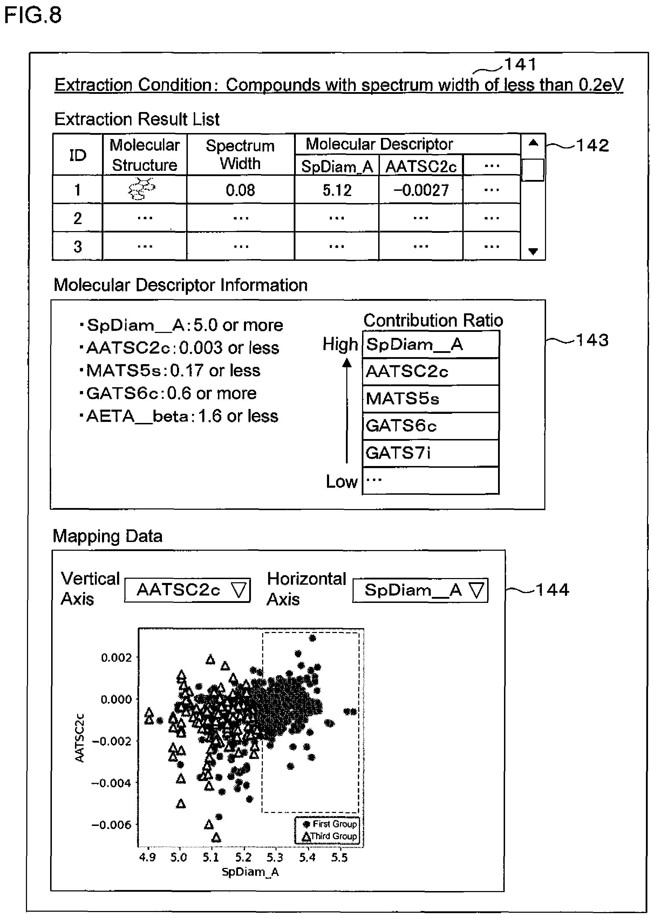

FIG. 8 is a schematic view illustrating an example of a result screen displayed on the display unit 14. The control unit 11 creates the result screen indicating the extraction result, on the basis of the obtained extraction result, and displays the result screen on the display unit 14. As illustrated in FIG. 8, in the result screen, for example, an extraction condition section 141, a result list 142, a molecular descriptor information section 143, a mapping data section 144, and the like are included.

In the extraction condition section 141, the extraction condition used for the extraction is displayed. The information processing device 1 displays the extraction condition received during the extraction in the extraction condition section 141.

In the result list 142, the extracted light emitting compound is displayed as a list. The information processing device 1 displays the molecular structure of each of the light emitting compounds extracted by the secondary extraction, the spectrum width by the quantum chemical calculation, the value for each of the molecular descriptors, and the like as a list, on the basis of the information stored in the candidate compound information.

In the molecular descriptor information section 143, the combination of the molecular descriptors and the range of the value of each of the molecular descriptors corresponding to the extraction condition are displayed. The information processing device 1 displays the combination of the molecular descriptors and the range of the value of each of the molecular descriptors, which are specified in step S25 of FIG. 5, in the molecular descriptor information section 143. In the molecular descriptor information section 143, information indicating the contribution ratio of the molecular descriptor is further displayed. The information processing device 1 displays the molecular descriptors side by side in descending order of the contribution ratio, in the molecular descriptor information section 143, on the basis of the contribution ratio of each of the molecular descriptors acquired in step S17 of FIG. 4.

In the mapping data section 144, the mapping data is displayed. The mapping data section 144 is configured such that the molecular descriptors on the vertical axis and the horizontal axis in the mapping data to be a display target can be changed in accordance with the manipulation of the user through the manipulation unit 15. The information processing device 1 reads out the mapping data including the molecular descriptor according to the selection of the user on the axis, among the plurality of pieces of mapping data created in step S24 of FIG. 5, and displays the mapping data in the mapping data section 144.

For example, a person responsible for synthesis of the light emitting compound is capable of easily grasping various types of information relevant to the light emitting compound to be the production candidate by using the result screen. The person responsible for synthesis is capable of determining the light emitting compound to be actually subjected to the synthesis, by checking the extraction result presented on the result screen.

According to the configuration described above, it is possible to efficiently extract the compound capable of satisfying a desired property, and perform the synthesis. The information processing device 1 is capable of accurately extracting the compound capable of satisfying a desired property, and reducing an arithmetic burden, by performing the quantum chemical calculation with respect to the compound subjected to the primary extraction by using the learning model 121.

According to the configuration described above, the condition of the molecular descriptor that is capable of extracting the compound satisfying a desired property is specified. By using the specified condition of the molecular descriptor, it is possible to efficiently extract the compound capable of satisfying a desired property from new candidate compounds. The condition of the molecular descriptor is efficiently specified by using the mapping data. As the condition of the molecular descriptor, conditions according to various extraction modes can be acquired by suitably adjusting the number of groupings for separating the compound group, a grouping condition, the value of the spectral indicator, and the like.

### (Modification Example)

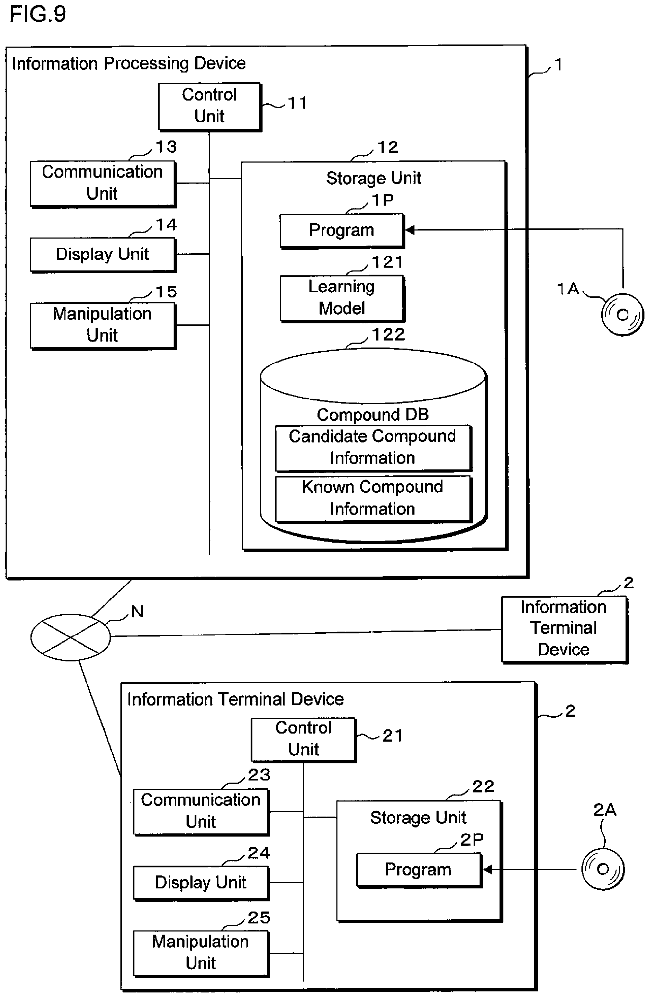

The information processing device 1 may be configured to provide the extraction result of the compound to an external device. FIG. 9 is a block diagram illustrating a configuration example of the information processing device 1 and the information terminal device 2 in the modification example. The information terminal device 2 is an example of the external device receiving the provision of the extraction result.

As illustrated in FIG. 9, the information processing device 1 is connected to each of a plurality of information terminal devices 2 through a network N such as the internet such that communication is available. The information processing device 1 and the information terminal device 2 are capable of transmitting and receiving data through the network N.

The information processing device 1 executes the extraction of the compound satisfying the extraction condition and the specifying of the descriptor, in accordance with the extraction condition received from the information terminal device 2, and transmits information indicating the execution result to the information terminal device 2. The information processing device 1 functions as a service providing server providing a compound extraction service.

The information terminal device 2, for example, is a personal computer, a smart phone, a tablet terminal, and the like, and is managed by the user using the extraction service.

The information terminal device 2 includes a control unit 21, a storage unit 22, a communication unit 23, a display unit 24, a manipulation unit 25, and the like. The storage unit 22 stores various computer programs and data, including a program 2P for causing a computer to execute processing relevant to the acquisition of the extraction result of the compound. Since the hardware configuration of the information terminal device 2 is the same as that of the information processing device 1, the details description will be omitted.

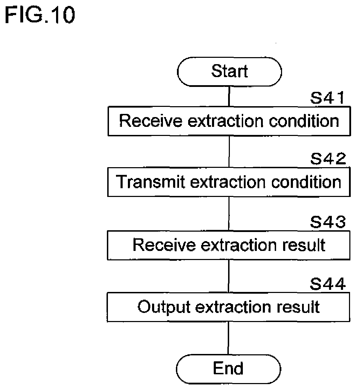

FIG. 10 is a flowchart illustrating an example of a processing procedure executed by the information terminal device 2. The processing in each of the following flowcharts may be executed by the control unit 21, in accordance with the program 2P stored in the storage unit 22 of the information terminal device 2, may be attained by a dedicated hardware circuit provided in the control unit 21, or may be attained by a combination thereof.

The control unit 21 of the information terminal device 2 receives the extraction condition by the user manipulating the manipulation unit 25 (step S41). The control unit 21, for example, receives the range of the spectrum width to be satisfied by the compound, as desired specifying for the compound to be the extraction target. The control unit 21 transmits the received extraction condition to the information processing device 1 (step S42). The control unit 21 may transmit identification information of the information terminal device 2 or the user in association with the extraction condition.

The information processing device 1 acquires the extraction condition transmitted from the information terminal device 2 by the processing of step S11 in FIG. 4. The information processing device 1 executes the processing illustrated in FIG. 4 and FIG. 5 to extract the compound satisfying the acquired extraction condition. In addition, the information processing device 1, for example, specifies the combination of the molecular descriptors and the range of the value of each of the molecular descriptors that are capable of separating the compound group satisfying the acquired extraction condition from the compound group not satisfying the extraction condition. The information processing device 1 transmits the extraction result to the information terminal device 2 by the processing of step S28 in FIG. 5. The information processing device 1 transmits the extraction result to the information terminal device 2 identified by the identification information of the information terminal device 2 or the user through the communication unit 13.

The control unit 21 of the information terminal device 2 receives the extraction result transmitted from the information processing device 1 (step S43). The control unit 21 outputs the received extraction result through the display unit 24 (step S44), and ends a series of processing. The display unit 24 displays the same result screen as that in FIG. 8.

According to the configuration described above, it is possible to improve the utility value of various types of information relevant to the extraction of the compound.

In this embodiment, it is possible to provide the light emitting compound extracted by the information processing method described above.

The light emitting compound satisfies SpDiam_A of 5.0 or more, AATSC2c of 0.003 or less, MATS5s of 0.17 or less, GATS6c of 0.6 or more, and AETA_beta of 1.6 or less in the molecular descriptor by mordred. By setting the molecular descriptor of the light emitting compound in the range described above, it is possible to make the light emitting spectrumgood, in particular, it is possible to narrow the spectrum width, and efficiently obtain such a light emitting compound.

It is preferable that the light emitting compound satisfies SpDiam_A of 5.0 or more and 6.0 or less, AATSC2c of -0.010 or more and 0.003 or less, MATS5s of - 0.30 or more and 0.17 or less, GATS6c of 0.6 or more and 3.0 or less, and AETA_beta of 0.5 or more and 1.6 or less. By setting the molecular descriptor of the light emitting compound in the range described above, it is possible to make the light emitting spectrum good, in particular, it is possible to narrow the spectrum width, and more efficiently obtain such a light emitting compound.

From the viewpoint of making the light emitting spectrum of the light emitting compound good, SpDiam_A is more preferably 5.7 or less, and even more preferably 5.5 or less. AATSC2c is more preferably -0.004 or more, and even more preferably -0.003 or more, and is more preferably 0.002 or less, and even more preferably 0.001 or less. MATS5s is more preferably -0.20 or more, and even more preferably -0.15 or more, and is more preferably 0.15 or less, and even more preferably 0.13 or less. GATS6c is more preferably 0.8 or more, and is more preferably 2.0 or less, and even more preferably 1.4 or less. AETA_beta is more preferably 1.0 or more, and even more preferably 1.2 or more.

It is more preferable that the light emitting compound further satisfies nB of 0 or more and 2 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, n6ARing of 0 or more and 10 or less, nARing of 0 or more and 10 or less, AXp-3d of 0.10 or more and 0.20 or less, SaasC of 0 or more and 40 or less, and Vabc of 100 or more and 2000 or less. By setting the molecular descriptor of the light emitting compound in the range described above, it is possible to make the light emitting spectrum good, in particular, it is possible to narrow the spectrum width, and more efficiently obtain such a light emitting compound.

From the viewpoint of making the light emitting spectrum of the light emitting compound good, nB is even more preferably 1 or less, and most preferably 0. AATSC3s is even more preferably -0.1 or more, and most preferably 0.0 or more, and is even more preferably 0.4 or less. C3SP2 is even more preferably 4 or more, and is even more preferably 16 or less. AETA_beta_s is even more preferably 0.6 or more, is and even more preferably 0.7 or less. SlogP_VSA5 is even more preferably 100 or less. n5aRing is even more preferably 6 or less, and most preferably 3 or less. n6ARing is even more preferably 4 or less, and most preferably 2 or less. nARing is even more preferably 2 or more, and is even more preferably 6 or less, and most preferably 5 or less. AXp-3d is even more preferably 0.15 or more, and is even more preferably 0.17 or less, and most preferably 0.16 or less. SaasC is even more preferably 1 or more, and is even more preferably 20 or less, and most preferably 15 or less. Vabc is even more preferably 200 or more, and is even more preferably 1500 or less, and most preferably 1000 or less.

In the light emitting compound according to the embodiment, the spectral indicator is less than 0.2 eV, and is preferably 0.12 eV or less, and more preferably 0.08 eV or less.

Examples of the structural formulae of light emitting compounds A1 to A82 of the embodiment are shown in Table 1.

**[Table 1-1]**

| Compound | Structural Formula | Compound | Structural Formula | Compound | Structural Formula |
|---|---|---|---|---|---|
| A1 | 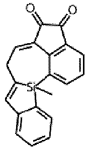 | A2 | 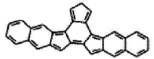 | A3 | 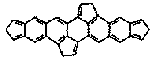 |
| A4 | 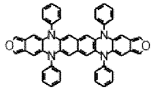 | A5 | 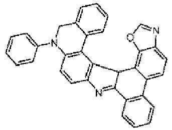 | A6 | 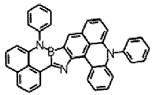 |
| A7 | 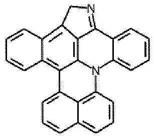 | A8 | 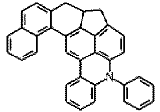 | A9 | 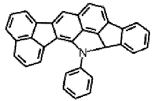 |
| A10 | 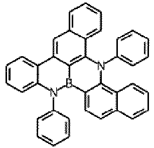 | A11 | 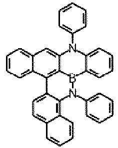 | A12 | 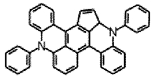 |
| A13 | 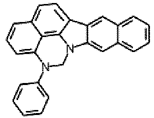 | A14 | 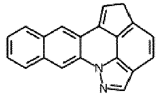 | A15 | 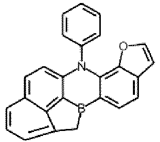 |
| A16 | 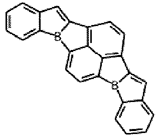 | A17 | 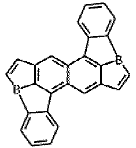 | A18 | 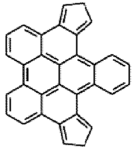 |
| A19 | 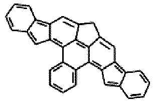 | A20 | 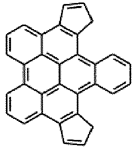 | A21 | 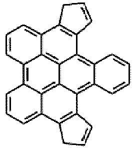 |

**[Table 1-2]**

| Compound | Structural Formula | Compound | Structural Formula | Compound | Structural Formula |
|---|---|---|---|---|---|
| A22 | 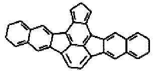 | A23 | 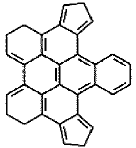 | A24 | 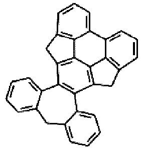 |
| A25 | 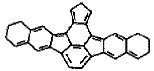 | A26 | 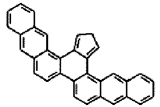 | A27 | 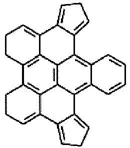 |
| A28 | 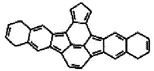 | A29 | 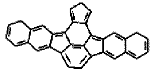 | A30 | 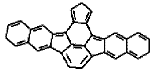 |
| A31 | 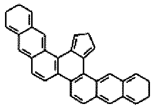 | A32 | 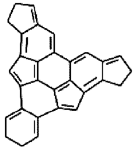 | A33 | 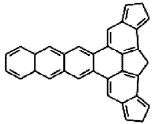 |
| A34 | 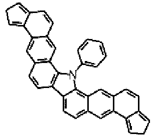 | A35 | 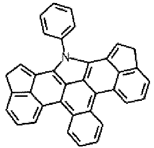 | A36 | 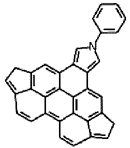 |
| A37 | 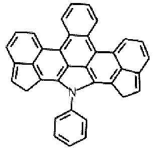 | A38 | 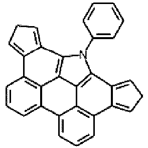 | A39 | 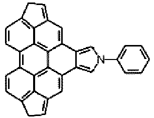 |
| A40 | 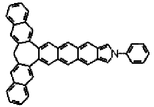 | A41 | 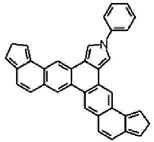 | A42 | 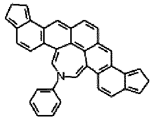 |

**[Table 1-3]**

| Compound | Structural Formula | Compound | Structural Formula | Compound | Structural Formula |
|---|---|---|---|---|---|
| A43 | 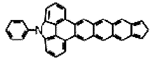 | A44 | 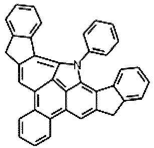 | A45 | 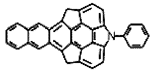 |
| A46 | 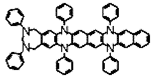 | A47 | 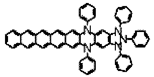 | A48 | 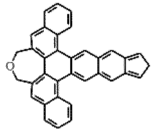 |
| A49 | 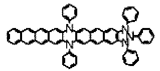 | A50 | 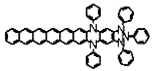 | A51 | 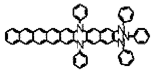 |
| A52 | 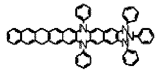 | A53 | 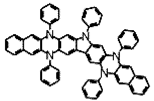 | A54 | 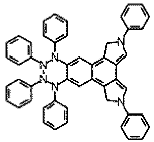 |
| A55 | 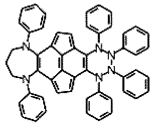 | A56 | 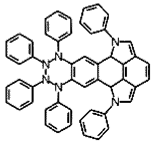 | A57 | 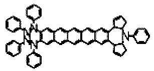 |
| A58 | 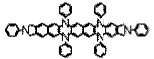 | A59 | 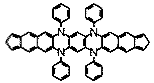 | A60 | 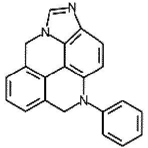 |
| A61 | 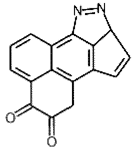 | A62 | 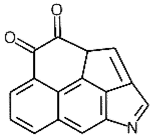 | A63 | 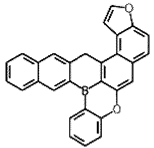 |

**[Table 1-4]**

| Compound | Structural Formula | Compound | Structural Formula | Compound | Structural Formula |
|---|---|---|---|---|---|
| A64 | 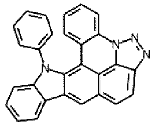 | A65 | 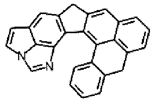 | A66 | 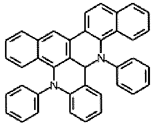 |
| A67 | 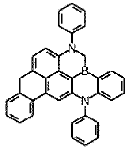 | A68 | 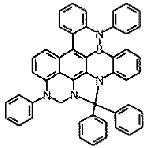 | A69 | 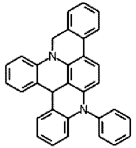 |
| A70 | 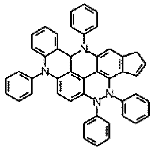 | A71 | 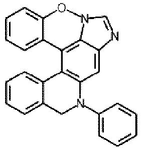 | A72 | 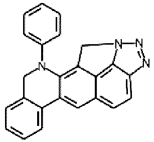 |
| A73 | 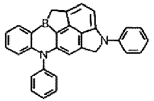 | A74 | 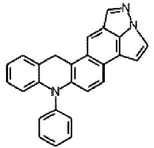 | A75 | 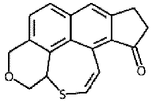 |
| A76 | 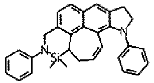 | A77 | 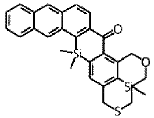 | A78 | 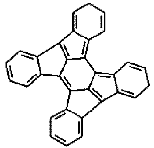 |
| A79 | 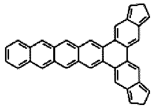 | A80 | 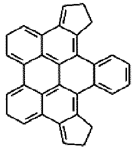 | A81 | 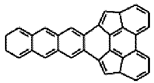 |
| A82 | 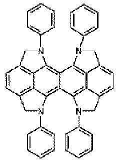 | | | | |

In this embodiment, a method for producing a light emitting compound to which the information processing method described above is applied can be provided.

The method for producing the light emitting compound includes a preparation step of preparing a plurality of compounds, and an extraction step of extracting a light emitting compound, from the plurality of compounds obtained in the preparation step.

The preparation step corresponds to a step of generating the plurality of compounds on a computer.

In the extraction step, the light emitting compound satisfying at least one of SpDiam_A of 5.0 or more, AATSC2c of 0.003 or less, MATS5s of 0.17 or less, GATS6c of 0.6 or more, AETA_beta of 1.6 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, and n6ARing of 0 or more and 10 or less in a molecular descriptor by mordred is extracted. The extraction step can be performed by extracting the light emitting compound by applying the information processing method described above.

A method for producing a light emitting compound of another aspect provided by this embodiment includes a preparation step of preparing a plurality of compounds, and an extraction step of extracting a light emitting compound in which the half width of a light emitting spectrum is less than a predetermined value, from the plurality of compounds obtained in the preparation step.

The preparation step corresponds to the step of generating the plurality of compounds on the computer.

In the extraction step, the light emitting compound satisfying at least one of SpDiam_A of 5.0 or more, AATSC2c of 0.003 or less, MATS5s of 0.17 or less, GATS6c of 0.6 or more, AETA_beta of 1.6 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, and n6ARing of 0 or more and 10 or less in a molecular descriptor by mordred is extracted. The extraction step can be performed by extracting the light emitting compound by applying the information processing method described above.

A method for producing a light emitting compound of another aspect provided by this embodiment includes a step of extracting a light emitting compound by the information processing method of the embodiment, and a step of obtaining the extracted light emitting compound.

In the step of obtaining the light emitting compound, for example, the light emitting compound can be obtained by a combination of a reaction such as a coupling reaction, an amination reaction, and a condensation reaction, a functional group conversion reaction such as a halogenation reaction, and the like.

**In** this embodiment, a composition containing the light emitting compound described above can be provided.

The composition contains the light emitting compound of the embodiment, and at least one type selected from the group consisting of a hole transport material, a hole injection material, an electron transport material, an electron injection material, a light emitting material, an antioxidant, and a solvent. Only one type of the light emitting compounds of the embodiment may be used alone, or two or more types thereof may be used in combination.

The hole transport material may be a low-molecular-weight compound or a high-molecular-weight compound. As the hole transport material, the high-molecular-weight compound is preferable, and examples thereof include polyvinyl carbazole and a derivative thereof, and polyarylene having an aromatic amine structure on a side chain or a main chain and a derivative thereof. Only one type of the hole transport materials may be used alone, or two or more types thereof may be used in combination.

The electron transport material may be a low-molecular-weight compound or a high-molecular-weight compound. The electron transport material may have a cross-linking group. Examples of the low-molecular-weight compound include a metal complex having 8-hydroxyquinoline as a ligand, oxadiazole, anthraquinodimethane, benzoquinone, naphthoquinone, anthraquinone, tetracyanoanthraquinodimethane, fluorenone, diphenyl dicyanoethylene, diphenoquinone, and derivatives thereof. Examples of the high-molecular-weight compound include polyphenylene, polyfluorene, and derivatives thereof. The high-molecular-weight compound may be doped with a metal. Only one type of the electron transport materials may be used alone, or two or more types thereof may be used in combination.

The hole injection material and the electron injection material may be a low-molecular-weight compound or a high-molecular-weight compound. The hole injection material and the electron injection material may have a cross-linking group. Examples of the low-molecular-weight compound include metal phthalocyanine such as copper phthalocyanine, a metal oxide of molybdenum, tungsten, and the like, a metal fluoride such as lithium fluoride, sodium fluoride, cesium fluoride, and potassium fluoride, carbon, and the like. Examples of the high-molecular-weight compound include polyaniline, polythiophene, polypyrrole, polyphenylene vinylene, polythienylene vinylene, polyquinoline, polyquinoxaline, and derivatives thereof, a conductive polymer such as a polymer having an aromatic amine structure on a main chain or a side chain, and the like.

The light emitting material may be a low-molecular-weight compound or a high-molecular-weight compound. The light emitting material may have a cross-linking group. Examples of the low-molecular-weight compound include naphthalene and a derivative thereof, anthracene and a derivative thereof, perylenen and a derivative thereof, a triplet light emitting complex having indium, platinum, or europium as a central metal, and the like. Examples of the high-molecular-weight compound include a high-molecular-weight compound having an arylene group such as a phenylene group, a naphthalene diyl group, a fluorene diyl group, a phenanthrene diyl group, a dihydrophenanthrene diyl group, an anthracene diyl group, and a pyrene diyl group, an aromatic amine residue such as a group obtained by removing two hydrogen atoms from aromatic amine, and a divalent heterocyclic group such as a carbazole diyl group, a phenoxazine diyl group, and a phenothiazine diyl group, and the like. Only one type of the light emitting materials may be used alone, or two or more types thereof may be used in combination.

The antioxidant is not particularly limited insofar as the antioxidant is a compound that does not inhibit light emission and charge transport, and examples thereof include a phenolic antioxidant, a phosphorus-based antioxidant, and the like. Only one type of the antioxidants may be used alone, or two or more types thereof may be used in combination.

Examples of the solvent include a chlorine-based solvent, an ether-based solvent, an aromatic hydrocarbon-based solvent, an aliphatic hydrocarbon-based solvent, a ketone-based solvent, an ester-based solvent, a polyhydric alcohol-based solvent, an alcohol-based solvent, a sulfoxide-based solvent, an amide-based solvent, and the like. Only one type of the solvents may be used alone, or two or more types thereof may be used in combination.

A method for producing a composition of the embodiment includes a step of mixing the light emitting compound produced by the method for producing a light emitting compound described above with at least one type selected from the group consisting of a hole transport material, a hole injection material, an electron transport material, an electron injection material, a light emitting material, an antioxidant, and a solvent.

In this embodiment, a light emitting element containing the light emitting compound described above can be provided.

The light emitting element includes an anode, a cathode, and a light emitting layer that is provided between the anode and the cathode and contains the light emitting compound of the embodiment. Only one type of the light emitting compounds of the embodiment may be used alone, or two or more types thereof may be used in combination.

It is preferable that the light emitting layer further contains at least one type selected from the group consisting of a hole transport material, a hole injection material, an electron transport material, an electron injection material, a light emitting material, and an antioxidant.

Examples of material of the anode include a conductive metal oxide, and a translucent metal, and the material is preferably indium oxide, zinc oxide, tin oxide, indium tin oxide (ITO), and the like.

Examples of the material of the cathode include metals such as lithium, sodium, potassium, rubidium, cesium, beryllium, magnesium, calcium, strontium, barium, aluminum, zinc, and indium, and an alloy of two or more types thereof, an alloy of one or more types of the above metals and one or more types of silver, copper, manganese, titanium, cobalt, nickel, tungsten, and tin, and the like. Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy. The anode and the cathode may each have a laminated structure of two or more layers.

A method for producing a light emitting element of the embodiment includes a step of forming the light emitting layer by using the light emitting compound produced by the method for producing a light emitting compound described above. The light emitting layer, for example, can be formed by a dry method and a wet method. The light emitting layer, for example, may be formed by using a vacuum deposition method, an ink jet method, a spin coating method, or the like.

The light emitting element can be preferably used for a light emitting device such as a display device of a computer, a television, a mobile terminal, and the like. Examples

Hereinafter, the present invention will be described in more detail, on the basis of Examples and Comparative Examples, but it is not intended that the present invention is limited to Examples.

The information processing method of the present disclosure was applied to the compounds A1 to A82 shown in Table 1 described above and compounds B1 to B16 shown in Table 2 described below. On the basis of the value of the molecular descriptor and the spectrum width in each of the compounds, a light emitting compound having a goodt light emitting spectrum can be extracted. The value of the molecular descriptor and the value of the spectrum width (FWHM) in each of the compounds are shown in Table 3 and Table 4. The spectrum width was calculated by using quantum chemical calculation software Gaussian16.

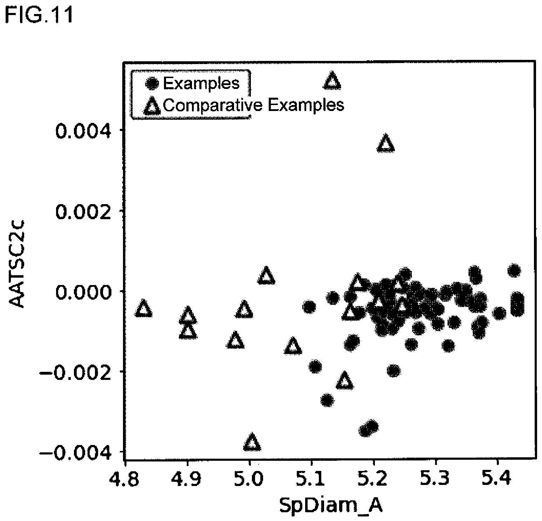

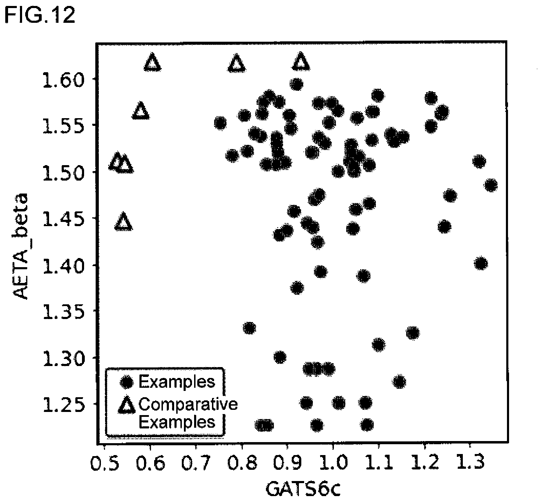

FIG. 11 and FIG. 12 are drawings illustrating mapping data of the compounds shown in Table 1 and Table 2. In FIG. 11 and FIG. 12, black circles indicate the compounds A1 to A82 (Examples) shown in Table 1, and black triangles indicate the compounds B1 to B16 (Comparative Examples) shown in Table 2.

FIG. 11 is mapping data including AATSC2c on the vertical axis of a graph, and SpDiam_A on the horizontal axis. FIG. 12 is mapping data including AETA_beta on the vertical axis of a graph, and GATS6c on the horizontal axis. The mapping data in FIG. 12 indicates mapping data in which, among the compounds A1 to A82 and the compounds B1 to B16, only the compound satisfying SpDiam_A of 5.0 or more, AATSC2c of 0.003 or less, and MATS5s of 0.17 or less as the condition of the molecular descriptor that has been already specified, is mapped.

The light emitting compounds of Examples are capable of having a spectrum width of less than 0.2 eV and a good light emitting spectrum.

Hereinafter, synthesis methods for some compounds will be described, but the other compounds can also be obtained by a combination of a reaction such as a coupling reaction, an amination reaction, and a condensation reaction, a functional group conversion reaction such as a halogenation reaction, and the like.

### Compound A8

The compound A8 is synthesized in accordance with the following synthesis scheme.

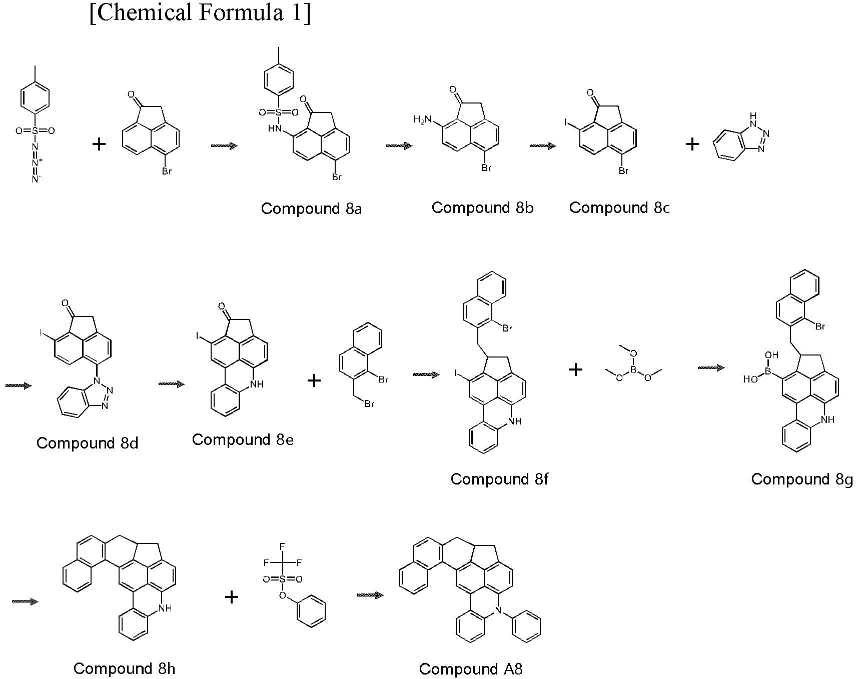

The compound 8a can be obtained by a reaction between p-methyl benzene sulfonyl azide and 5-bromo-1-acenaphthenone, in accordance with a procedure described in [Organic Letters, 2017, vol. 19, No. 10, p. 2502-2505] or the like. The compound 8b can be obtained by the reaction of the compound 8a, in accordance with a procedure described in [Yuki Gosei Kagaku Kyokaishi, 1959, vol. 17, p. 142, 143] or the like. The compound 8c can be obtained by the reaction of the compound 8b, in accordance with a procedure described in [Advanced Synthesis and Catalysis, 2018, vol. 360, No. 20, p. 3877-3883] or the like. The compound 8d can be obtained by a reaction between the compound 8c and 1,2,3-benzotriazole, in accordance with a procedure described in [Bioorganic and Medicinal Chemistry, 2011, vol. 19, No. 24, p. 7519-7525] or the like. The compound 8e can be obtained by the reaction of the compound 8d, in accordance with a procedure described in [European Journal of Organic Chemistry, 2017, vol. 2017, No. 22, p. 3197-3210] or the like. The compound 8f can be obtained by a reaction between the compound 8e and 1-bromo-2-(bromomethyl) naphthalene, in accordance with a procedure described in [Journal of Organic Chemistry, 1987, vol. 52, No. 19, p. 4207-4214] or the like. The compound 8g can be obtained by a reaction between the compound 8f and trimethyl borate, in accordance with a procedure described in [Journal of Fluorine Chemistry, 2005, vol. 126, No. 4, p. 483-490] or the like. The compound 8h can be obtained by the reaction of the compound 8g, in accordance with a procedure described in [Chemical Communications, 2011, vol. 47, No. 27, p. 7725-7727] or the like. The compound A8 can be obtained by a reaction between the compound 8h and phenyl triflate, in accordance with a procedure described in [Journal of the American Chemical Society, 2000, vol. 122, No. 10, p. 2178-2192] or the like.

### Compound A9

The compound A9 is synthesized in accordance with the following synthesis scheme.

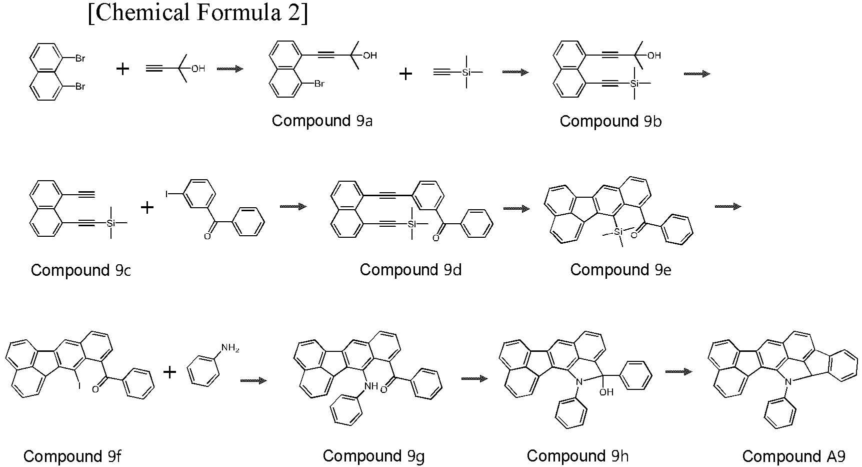

The compound 9a can be obtained by a reaction between 1,8-dibromonaphthalene and 3-methyl-1-butyn-3-ol, in accordance with a procedure described in [Beilstein Journal of Organic Chemistry, 2014, vol. 10, p. 384-393] or the like. The compound 9b can be obtained by a reaction between the compound 9a and trimethyl silyl acetylene, in accordance with a procedure described in [Tetrahedron Letters, 2016, vol. 57, No. 10, p. 1100-1103] or the like. The compound 9c can be obtained by the reaction of the compound 9b, in accordance with a procedure described in [Journal of the Chemical Society. Perkin Transactions 2 (2001), 2002, No. 5, p. 878-886] or the like. The compound 9d can be obtained by a reaction between the compound 9c and 3-iodobenzophenone, in accordance with a procedure described in [Tetrahedron, 2010, vol. 66, No. 13, p. 2378-2383] or the like. The compound 9e can be obtained by the reaction of the compound 9d, in accordance with a procedure described in [Journal of the American Chemical Society, 2021, vol. 143, No. 37, p. 15420-15426] or the like. The compound 9f can be obtained by the reaction of the compound 9e, in accordance with a procedure described in [Journal of Organic Chemistry, 2015, vol. 80, No. 19, p. 9410-9424] or the like. The compound 9g can be obtained by a reaction between the compound 9f and aniline, in accordance with a procedure described in [European Journal of Medicinal Chemistry, 2017, vol. 135, p. 1-11] or the like. The compound 9h can be obtained by the reaction of the compound 9g, in accordance with a procedure described in [Journal of Organic Chemistry USSR (English Translation), 1986, vol. 22, p. 199-200] or the like. The compound A9 can be obtained by the reaction of the compound 9h, in accordance with a procedure described in [Journal of the American Chemical Society, 1966, vol. 88, p. 1482-1488] or the like.

### Compound A13

The compound A13 is synthesized in accordance with the following synthesis scheme.

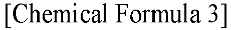

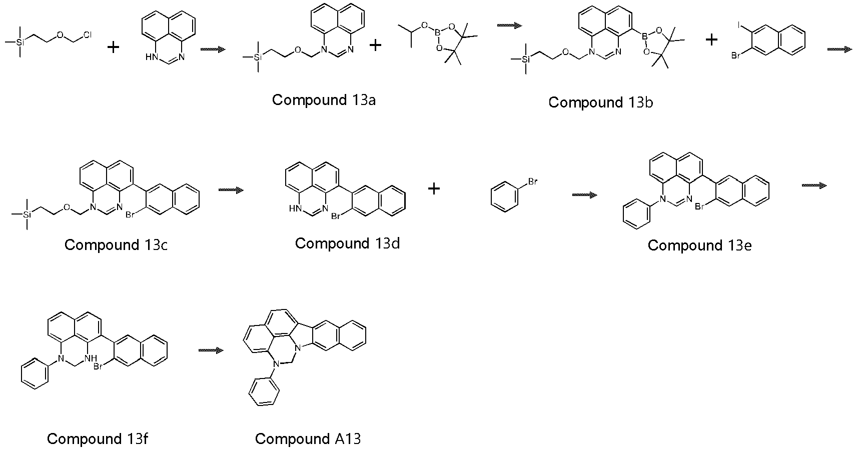

The compound 13a can be obtained by a reaction between 2-(chloromethoxy)ethyl trimethyl silane and 1H-perimidine, in accordance with a procedure described in [Journal of Organic Chemistry, 1986, vol. 51, No. 10, p. 1891-1894] or the like. The compound 13b can be obtained by a reaction between the compound 13a and 2-isopropoxy-4,4,5,5-tetramethyl-1,3,2-dioxaborolane, in accordance with a procedure described in [Organic Letters, 2011, vol. 13, No. 14, p. 3588-3591] or the like. The compound 13c can be obtained by a reaction between the compound 13b and 2-bromo-3-iodonaphthalene, in accordance with a procedure described in [Organic Letters, 2015, vol. 17, No. 6, p. 1613-1616] or the like. The compound 13d can be obtained by the reaction of the compound 13c, in accordance with a procedure described in [Tetrahedron Letters, 1993, vol. 34, No. 12, p. 1885-1888] or the like. The compound 13e can be obtained by a reaction between the compound 13d and bromobenzene, in accordance with a procedure described in [Organometallics, 2010, vol. 29, No. 18, p. 4120-4129] or the like. The compound 13f can be obtained by the reaction of the compound 13e, in accordance with a procedure described in [Pharmaceutical Chemistry Journal, 1982, vol. 16, No. 11, p. 844-848] or the like. The compound A13 can be obtained by the reaction of the compound 13f, in accordance with a procedure described in [Bioorganic and Medicinal Chemistry Letters, 2018, vol. 28, No. 18, p. 3123-3128] or the like.

### Compound A21

The compound A21 is synthesized in accordance with the following synthesis scheme.

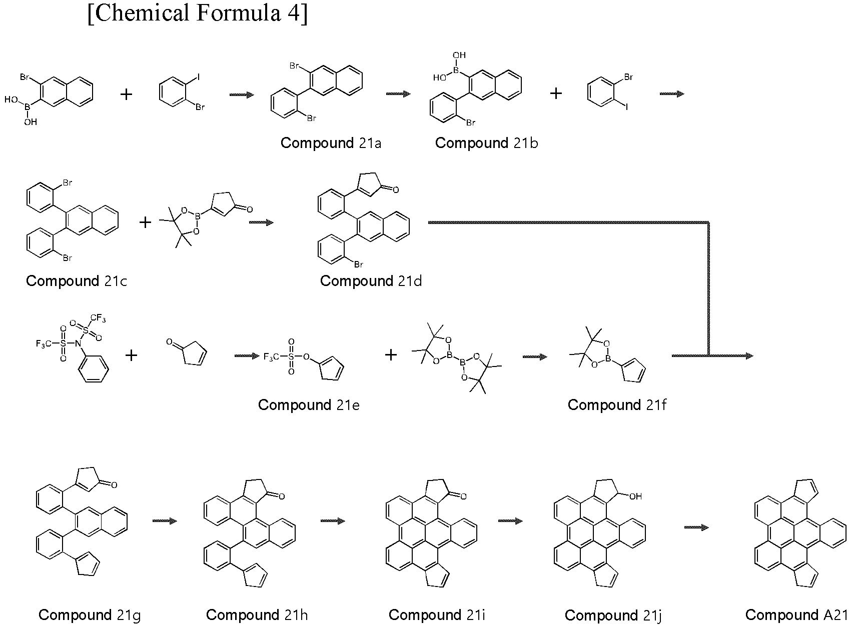

The compound 21a can be obtained by a reaction between a (3-bromonaphthalen-2-yl) boronic acid and 1-bromo-2-iodobenzene, in accordance with a procedure described in [Journal of Organic Chemistry, 2015, vol. 80, No. 15, p. 7779-7784] or the like. The compound 21b can be obtained by the reaction of the compound 21a, in accordance with a procedure described in [New Journal of Chemistry, 2008, vol. 32, No. 11, p. 1847-1849] or the like. The compound 21c can be obtained by a reaction between the compound 21b and 1-bromo-2-iodobenzene, in accordance with a procedure described in [Journal of Organic Chemistry, 2021, vol. 86, No. 24, p. 17651-17666] or the like. The compound 21d can be obtained by a reaction between the compound 21c and 3-(4,4,5,5-tetramethyl-1,3,2-dioxaborolan-2-yl) cyclopenta-2-en-1-one, in accordance with a procedure described in [Tetrahedron Letters, 2009, vol. 50, No. 5, p. 590-592] or the like. The compound 21e can be obtained by a reaction between N-phenyl bis(trifluoromethane sulfone imide) and 3-cyclopentenone, in accordance with a procedure described in [Journal of the American Chemical Society, 2018, vol. 140, No. 47, p. 16253-16263] or the like. The compound 21f can be obtained by a reaction between the compound 21e and bis(pinacolato)diboron, in accordance with a procedure described in [Bioorganic and Medicinal Chemistry Letters, 2021, vol. 36, art. no. 127823] or the like. The compound 21g can be obtained by a reaction between the compound 21d and the compound 21f, in accordance with a procedure described in [Tetrahedron Letters, 2013, vol. 54, No. 6, p. 512-514] or the like. The compound 21h can be obtained by the reaction of the compound 21g, in accordance with a procedure described in [Organic Letters, 2016, vol. 18, No. 2, p. 200-203] or the like. The compound 21i can be obtained by the reaction of the compound 21h, in accordance with a procedure described in [Chemical Science, 2019, vol. 10, No. 14, p. 4025-4031] or the like. The compound 21j can be obtained by the reaction of the compound 21i, in accordance with a procedure described in [Chemistry-A European Journal, 2015, vol. 21, No. 33, p. 11813-11824] or the like. The compound A21 can be obtained by the reaction of the compound 21j, in accordance with a procedure described in [Journal of Organometallic Chemistry, 1992, vol. 426, No. 2, p. 213-245] or the like.

### Compound A60

The compound A60 is synthesized in accordance with the following synthesis scheme.

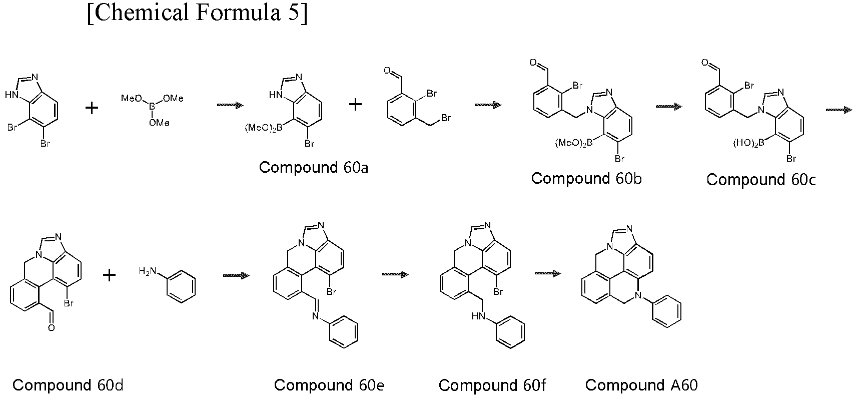

The compound 60a can be obtained by a reaction between 6,7-dibromo-1H-benzoimidazole and trimethyl borate, in accordance with a procedure described in [Dalton Transactions, 2009, No. 40, p. 8667-8682] or the like. The compound 60b can be obtained by a reaction between the compound 60a and 2-bromo-3-(bromomethyl) benzaldehyde, in accordance with a procedure described in [MedChemComm, 2013, vol. 4, No. 1, p. 140-144] or the like. The compound 60c can be obtained by the reaction of the compound 60b, in accordance with a procedure described in [Applied Organometallic Chemistry, 2012, vol. 26, No. 6, p. 287-292] or the like. The compound 60d can be obtained by the reaction of the compound 60c, in accordance with a procedure described in [Tetrahedron, 2008, vol. 64, No. 46, p. 10573-10580] or the like. The compound 60e can be obtained by a reaction between the compound 60d and aniline, in accordance with a procedure described in [Journal of the Chemical Society, 1957, p. 2210, 2212] or the like. The compound 60f can be obtained by the reaction of the compound 60e, in accordance with a procedure described in [Journal of Polymer Science, Part A: Polymer Chemistry, 2014, vol. 52, No. 21, p. 3096-3106] or the like. The compound A60 can be obtained by the reaction of the compound 60f, in accordance with a procedure described in [Journal of Organic Chemistry, 2012, vol. 77, No. 20, p. 9418-9421, 4] or the like.

### Compound A66

The compound A66 is synthesized in accordance with the following synthesis scheme.

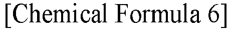

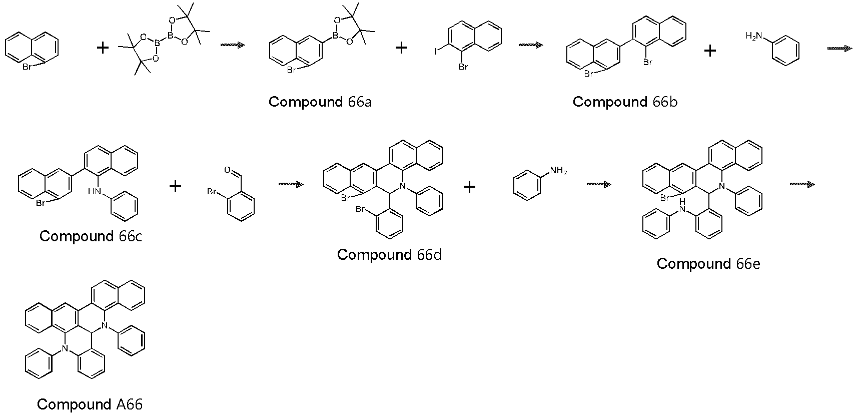

The compound 66a can be obtained by a reaction between 1-bromonaphthalene and bis(pinacolato)diboron, in accordance with a procedure described in [Angewandte Chemie-International Edition, 2018, vol. 57, No. 48, p. 15762-15766] or the like. The compound 66b can be obtained by a reaction between the compound 66a and 1-bromo-2-iodonaphthalene, in accordance with a procedure described in [Spectrochimica Acta Part A: Molecular and Biomolecular Spectroscopy, 2018, vol. 194, p. 111-116] or the like. The compound 66c can be obtained by a reaction between the compound 66b and aniline, in accordance with a procedure described in [Journal of Organic Chemistry, 2013, vol. 78, No. 10, p. 4649-4664] or the like. The compound 66d can be obtained by a reaction between the compound 66c and 2-bromobenzaldehyde, in accordance with a procedure described in [Tetrahedron Letters, 2013, vol. 54, No. 46, p. 6171-6177] or the like. The compound 66e can be obtained by a reaction between the compound 66d and aniline, in accordance with a procedure described in [Bioorganic and Medicinal Chemistry, 2016, vol. 24, No. 19, p. 4675-4691] or the like. The compound A66 can be obtained by the reaction of the compound 66e, in accordance with a procedure described in [Organic Letters, 2018, vol. 20, No. 4, p. 958-961] or the like.

### Compound A70

The compound A70 is synthesized in accordance with the following synthesis scheme.

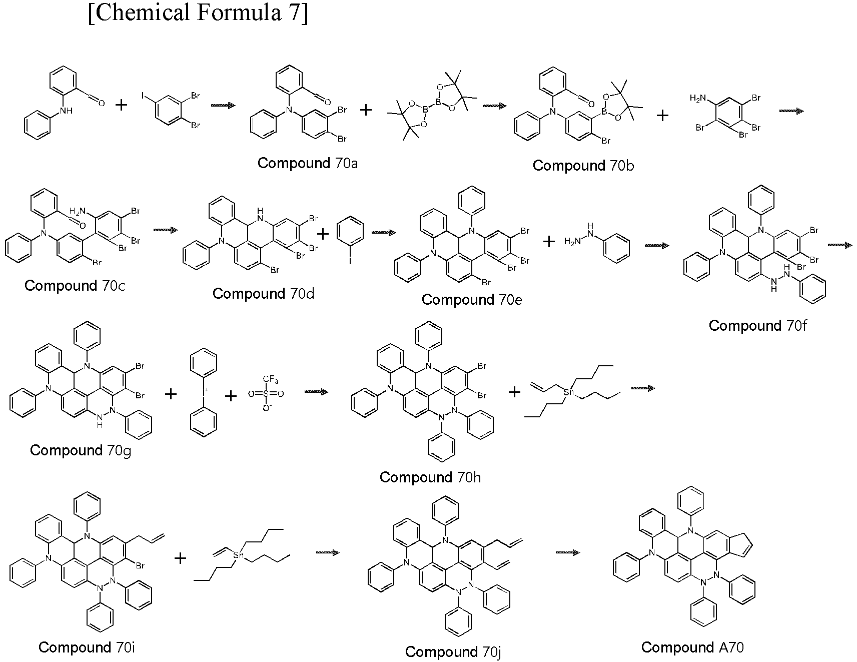

The compound 70a can be obtained by a reaction between 2-((N-phenyl) amino)benzaldehyde and 1,2-dibromo-4-iodobenzene, in accordance with a procedure described in [Chemistry-A European Journal, 2018, vol. 24, No. 18, p. 4519-4522] or the like. The compound 70b can be obtained by a reaction between the compound 70a and bis(pinacolato)diboron, in accordance with a procedure described in [European Journal of Medicinal Chemistry, 2017, vol. 137, p. 139-155] or the like. The compound 70c can be obtained by a reaction between the compound 70b and 2,3,4,5-tetrabromoaniline, in accordance with a procedure described in [Journal of Medicinal Chemistry, 2006, vol. 49, No. 1, p. 35-38] or the like. The compound 70d can be obtained by the reaction of the compound 70c, in accordance with a procedure described in [Il Farmaco, 1990, vol. 45, No. 1, p. 7-27] or the like. The compound 70e can be obtained by a reaction between the compound 70d and iodobenzene, in accordance with a procedure described in [Advanced Synthesis and Catalysis, 2014, vol. 356, No. 18, p. 3821-3830] or the like. The compound 70f can be obtained by a reaction between the compound 70e and phenyl hydrazine, in accordance with a procedure described in [Journal of the Indian Chemical Society, 1957, vol. 34, p. 77] or the like. The compound 70g can be obtained by the reaction of the compound 70f, in accordance with a procedure described in [European Journal of Medicinal Chemistry, 2012, vol. 58, p. 214-227] or the like. The compound 70h can be obtained by a reaction between the compound 70g, diphenyl iodonium, and a trifluoromethane sulfonic acid, in accordance with a procedure described in [Angewandte Chemie-International Edition, 2018, vol. 57, No. 35, p. 11427-11431] or the like. The compound 70i can be obtained by a reaction between the compound 70h and allyl tributyl tin, in accordance with a procedure described in [Angewandte Chemie-International Edition, 2017, vol. 56, No. 21, p. 5886-5889] or the like. The compound 70j can be obtained by a reaction between the compound 70i and tributyl vinyl tin, in accordance with a procedure described in [Organic Letters, 2018, vol. 20, No. 18, p. 5680-5683] or the like. The compound A70 can be obtained by the reaction of the compound 70j, in accordance with a procedure described in [Chemistry-A European Journal, 2020, vol. 26, No. 8, p. 1772-1775] or the like.

### Compound A72

The compound A72 is synthesized in accordance with the following synthesis scheme.

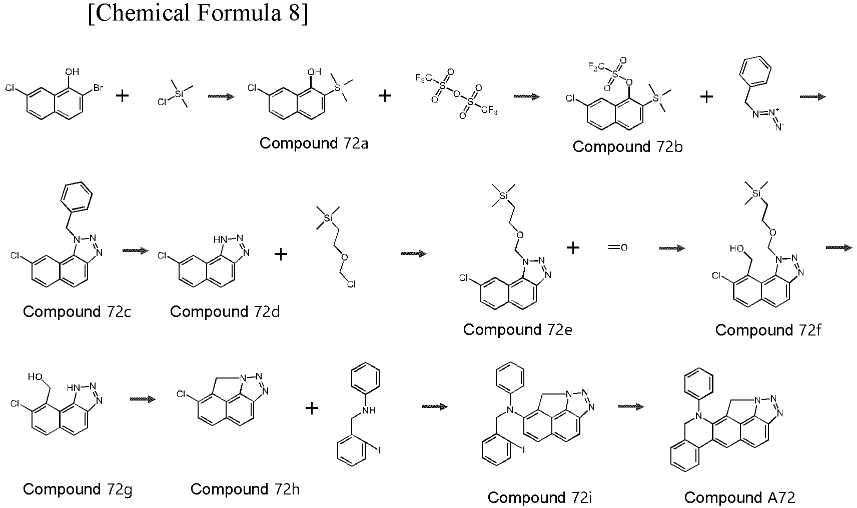

The compound 72a can be obtained by a reaction between 2-bromo-7-chloronaphthalen-1-ol and chlorotrimethyl silane, in accordance with a procedure described in [Tetrahedron Letters, 1995, vol. 36, No. 46, p. 8415-8418] or the like. The compound 72b can be obtained by a reaction between the compound 72a and a trifluoromethane sulfonic anhydride, in accordance with a procedure described in [Journal of Organic Chemistry, 2017, vol. 82, No. 8, p. 4242-4253] or the like. The compound 72c can be obtained by a reaction between the compound 72b and benzyl azide, in accordance with a procedure described in [Chemical Science, 2016, vol. 7, No. 8, p. 5206-5211] or the like. The compound 72d can be obtained by the reaction of the compound 72c, in accordance with a procedure described in [Monatshefte fur Chemie, 2010, vol. 141, No. 7, p. 773-779] or the like. The compound 72e can be obtained by a reaction between the compound 72d and 2-(chloromethoxy)ethyl trimethyl silane, in accordance with a procedure described in [Bioorganic and Medicinal Chemistry Letters, 1996, vol. 6, No. 24, p. 2919-2924] or the like. The compound 72f can be obtained by a reaction between the compound 72e and formaldehyde, in accordance with a procedure described in [Journal of the American Chemical Society, 2019] or the like. The compound 72g can be obtained by the reaction of the compound 72f, in accordance with a procedure described in [Journal of Medicinal Chemistry, 2021, vol. 64, No. 1, p. 695-710] or the like. The compound 72h can be obtained by the reaction of the compound 72g, in accordance with a procedure described in [Indian Journal of Chemistry-Section B Organic and Medicinal Chemistry, 1984, vol. 23, No. 9, p. 844-848] or the like. The compound 72i can be obtained by a reaction between the compound 72h and N-(2-iodophenylmethyl) aniline N-(2-iodophenyl methyl) aniline, in accordance with a procedure described in [Memorial des services chimiques de l'Etat, 1946, vol. 32, p. 62, 66] or the like. The compound A72 can be obtained by the reaction of the compound 72i, in accordance with a procedure described in [Tetrahedron, 2009, vol. 65, No. 17, p. 3409-3416] or the like.

**[Table 2]**

| Compound | Structural Formula | Compound | Structural Formula | Compound | Structural Formula |
|---|---|---|---|---|---|
| B1 | 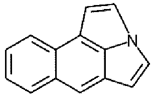 | B2 | 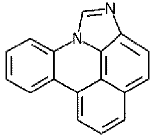 | B3 | 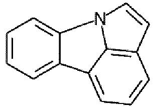 |
| B4 | 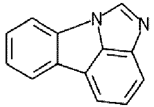 | B5 | 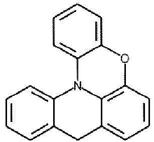 | B6 | 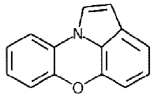 |
| B7 | 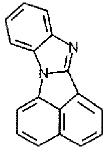 | B8 | 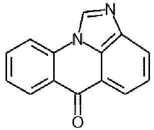 | B9 | 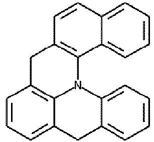 |
| B10 | 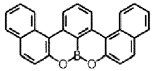 | B11 | 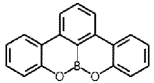 | B12 | 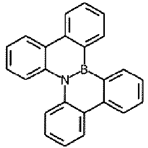 |
| B13 | 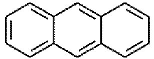 | B14 | 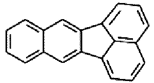 | B15 | 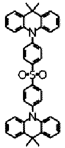 |
| B16 | 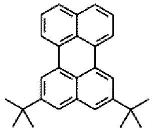 | | | | |

**[Table 3-1]**

| Compound | Molecular Descriptor | | | | | | | | | | | | | | | | Spectrum Width FWHM [eV] |
|---|---|---|---|---|---|---|---|---|---|---|---|---|---|---|---|---|---|
| | SpDiam_A | AATSC2c | AATSC3s | MATS5s | GATS6c | C3SP2 | AETA_beta | AETA_beta_s | SlogP_VSA5 | n5aRing | n6ARing | nB | nARing | AXp-3d | SaasC | Vabc | |
| A1 | 5.12 | -0.0027 | 0.37 | -0.11 | 0.97 | 4 | 1.39 | 0.652 | 28 | 0 | 0 | 0 | 3 | 0.15 | 5.4 | 298 | 0.08 |
| A2 | 5.13 | -0.0002 | 0.00 | 0.05 | 1.06 | 14 | 1.39 | 0.613 | 18 | 0 | 0 | 0 | 3 | 0.15 | 2.8 | 352 | 0.08 |
| A3 | 5.24 | 0.0001 | 0.00 | -0.02 | 1.10 | 16 | 1.31 | 0.625 | 24 | 0 | 0 | 0 | 4 | 0.15 | 3.1 | 359 | 0.08 |
| A4 | 5.26 | -0.0014 | 0.00 | 0.07 | 0.84 | 6 | 1.56 | 0.670 | 0 | 2 | 0 | 0 | 0 | 0.16 | 4.3 | 590 | 0.08 |
| A5 | 5.25 | -0.0005 | 0.00 | 0.08 | 0.91 | 8 | 1.55 | 0.664 | 28 | 1 | 2 | 0 | 3 | 0.15 | 13.3 | 417 | 0.11 |
| A6 | 5.29 | -0.0006 | 0.01 | 0.09 | 1.09 | 7 | 1.58 | 0.663 | 6 | 0 | 2 | 1 | 3 | 0.15 | 12.0 | 499 | 0.09 |
| A7 | 5.37 | -0.0009 | 0.02 | -0.08 | 0.88 | 9 | 1.58 | 0.658 | 17 | 0 | 2 | 0 | 3 | 0.14 | 10.4 | 325 | 0.11 |
| A8 | 5.31 | -0.0001 | 0.00 | -0.07 | 0.88 | 11 | 1.52 | 0.632 | 23 | 0 | 2 | 0 | 3 | 0.15 | 14.0 | 406 | 0.12 |
| A9 | 5.23 | -0.0005 | 0.00 | 0.00 | 0.86 | 12 | 1.58 | 0.640 | 17 | 1 | 0 | 0 | 2 | 0.14 | 13.8 | 361 | 0.12 |
| A10 | 5.36 | 0.0003 | 0.01 | 0.07 | 0.97 | 6 | 1.57 | 0.646 | 0 | 0 | 2 | 1 | 2 | 0.15 | 11.4 | 486 | 0.11 |
| A11 | 5.36 | 0.0004 | 0.00 | 0.06 | 1.00 | 6 | 1.57 | 0.646 | 0 | 0 | 2 | 1 | 2 | 0.15 | 11.4 | 486 | 0.10 |
| A12 | 5.35 | 0.0000 | 0.00 | 0.04 | 0.84 | 8 | 1.54 | 0.641 | 17 | 0 | 2 | 0 | 3 | 0.15 | 14.2 | 446 | 0.11 |
| A13 | 5.18 | 0.0002 | 0.00 | 0.00 | 0.99 | 6 | 1.55 | 0.655 | 0 | 1 | 1 | 0 | 1 | 0.15 | 2.5 | 311 | 0.11 |
| A14 | 5.17 | -0.0013 | 0.02 | -0.02 | 1.04 | 7 | 1.51 | 0.648 | 6 | 1 | 0 | 0 | 1 | 0.15 | 1.4 | 221 | 0.09 |
| A15 | 5.23 | -0.0010 | 0.00 | 0.02 | 1.15 | 4 | 1.54 | 0.679 | 6 | 1 | 1 | 1 | 2 | 0.15 | 7.9 | 324 | 0.10 |
| A16 | 5.16 | -0.0014 | 0.01 | 0.01 | 0.87 | 6 | 1.54 | 0.679 | 22 | 0 | 0 | 2 | 4 | 0.14 | 11.6 | 353 | 0.10 |
| A17 | 5.21 | -0.0006 | 0.01 | 0.06 | 0.97 | 8 | 1.54 | 0.679 | 11 | 0 | 0 | 2 | 4 | 0.14 | 14.6 | 353 | 0.09 |
| A18 | 5.43 | -0.0004 | 0.02 | -0.05 | 1.04 | 16 | 1.44 | 0.625 | 13 | 0 | 0 | 0 | 2 | 0.14 | 0.0 | 335 | 0.10 |
| A19 | 5.28 | -0.0004 | 0.00 | -0.01 | 0.96 | 16 | 1.47 | 0.621 | 22 | 0 | 0 | 0 | 3 | 0.15 | 5.8 | 361 | 0.08 |
| A20 | 5.43 | -0.0005 | 0.02 | -0.07 | 1.01 | 16 | 1.50 | 0.625 | 22 | 0 | 0 | 0 | 2 | 0.14 | 5.9 | 335 | 0.11 |
| A21 | 5.43 | -0.0005 | 0.01 | -0.04 | 1.05 | 16 | 1.50 | 0.625 | 22 | 0 | 0 | 0 | 2 | 0.14 | 5.9 | 335 | 0.11 |
| A22 | 5.21 | -0.0001 | 0.02 | 0.05 | 0.99 | 16 | 1.29 | 0.621 | 32 | 2 | 2 | 0 | 3 | 0.15 | 11.6 | 366 | 0.09 |
| A23 | 5.43 | -0.0002 | 0.02 | -0.06 | 1.01 | 16 | 1.25 | 0.625 | 37 | 0 | 2 | 0 | 4 | 0.14 | 3.2 | 362 | 0.10 |
| A24 | 5.30 | -0.0005 | -0.01 | 0.00 | 0.98 | 16 | 1.53 | 0.621 | 33 | 0 | 0 | 0 | 3 | 0.14 | 14.9 | 363 | 0.09 |
| A25 | 5.21 | -0.0001 | 0.03 | 0.07 | 1.07 | 16 | 1.23 | 0.621 | 43 | 2 | 2 | 0 | 3 | 0.15 | 8.9 | 368 | 0.10 |
| A26 | 5.22 | -0.0003 | 0.01 | 0.02 | 1.05 | 14 | 1.52 | 0.606 | 6 | 0 | 0 | 0 | 1 | 0.16 | 0.0 | 362 | 0.10 |
| A27 | 5.43 | -0.0003 | 0.02 | -0.09 | 1.01 | 16 | 1.25 | 0.625 | 37 | 0 | 2 | 0 | 4 | 0.14 | 3.3 | 362 | 0.12 |
| A28 | 5.21 | -0.0004 | 0.01 | 0.06 | 0.96 | 16 | 1.23 | 0.621 | 29 | 2 | 2 | 0 | 3 | 0.15 | 6.0 | 366 | 0.08 |
| A29 | 5.21 | -0.0003 | 0.01 | 0.05 | 0.96 | 16 | 1.29 | 0.621 | 18 | 2 | 2 | 0 | 3 | 0.15 | 8.6 | 363 | 0.09 |
| A30 | 5.21 | -0.0002 | 0.02 | 0.04 | 0.95 | 16 | 1.29 | 0.621 | 30 | 2 | 2 | 0 | 3 | 0.15 | 8.4 | 363 | 0.10 |

**[Table 3-2]**

| Compound | Molecular Descriptor | | | | | | | | | | | | | | | | Spectrum Width FWHM [eV] |
|---|---|---|---|---|---|---|---|---|---|---|---|---|---|---|---|---|---|
| | SpDiam_A | AATSC2c | AATSC3s | MATS5s | GATS6c | C3SP2 | AETA_beta | AETA_beta_s | SlogP_VSA5 | n5aRing | n6ARing | nB | nARing | AXp-3d | SaasC | Vabc | |
| A31 | 5.22 | -0.0001 | 0.02 | 0.02 | 1.14 | 14 | 1.27 | 0.606 | 32 | 0 | 2 | 0 | 3 | 0.16 | 0.0 | 389 | 0.09 |
| A32 | 5.32 | -0.0001 | 0.02 | -0.07 | 0.88 | 16 | 1.30 | 0.633 | 37 | 2 | 1 | 0 | 3 | 0.14 | 3.2 | 319 | 0.09 |
| A33 | 5.27 | -0.0001 | 0.00 | 0.07 | 0.84 | 14 | 1.23 | 0.621 | 24 | 0 | 2 | 0 | 5 | 0.15 | 3.1 | 388 | 0.08 |
| A34 | 5.16 | -0.0002 | 0.02 | 0.03 | 0.91 | 14 | 1.46 | 0.628 | 13 | 1 | 0 | 0 | 2 | 0.15 | 1.2 | 451 | 0.08 |
| A35 | 5.37 | -0.0004 | 0.01 | 0.02 | 1.08 | 14 | 1.51 | 0.642 | 11 | 1 | 0 | 0 | 2 | 0.14 | 4.1 | 387 | 0.10 |
| A36 | 5.40 | -0.0006 | 0.01 | -0.02 | 0.87 | 16 | 1.51 | 0.650 | 11 | 1 | 0 | 0 | 2 | 0.14 | 4.2 | 355 | 0.12 |
| A37 | 5.37 | -0.0002 | 0.01 | 0.00 | 1.04 | 14 | 1.51 | 0.642 | 11 | 1 | 0 | 0 | 2 | 0.14 | 4.2 | 387 | 0.10 |
| A38 | 5.36 | -0.0003 | 0.02 | -0.04 | 0.88 | 12 | 1.43 | 0.644 | 13 | 1 | 0 | 0 | 2 | 0.14 | 1.2 | 346 | 0.10 |
| A39 | 5.40 | -0.0006 | 0.00 | 0.01 | 0.85 | 16 | 1.51 | 0.650 | 11 | 1 | 0 | 0 | 2 | 0.14 | 4.2 | 355 | 0.09 |
| A40 | 5.10 | -0.0004 | -0.01 | 0.06 | 0.85 | 16 | 1.57 | 0.619 | 11 | 1 | 0 | 0 | 1 | 0.16 | 9.3 | 486 | 0.09 |
| A41 | 5.20 | -0.0005 | 0.01 | -0.06 | 0.95 | 14 | 1.44 | 0.628 | 13 | 1 | 0 | 0 | 2 | 0.16 | 1.2 | 410 | 0.09 |
| A42 | 5.21 | -0.0005 | 0.02 | 0.01 | 1.24 | 14 | 1.44 | 0.628 | 13 | 0 | 0 | 0 | 2 | 0.15 | 1.2 | 410 | 0.08 |
| A43 | 5.23 | 0.0001 | 0.00 | 0.06 | 0.95 | 12 | 1.52 | 0.632 | 6 | 1 | 0 | 0 | 1 | 0.15 | 1.2 | 382 | 0.08 |
| A44 | 5.30 | -0.0005 | 0.00 | 0.02 | 1.05 | 14 | 1.56 | 0.635 | 22 | 1 | 0 | 0 | 2 | 0.15 | 12.6 | 419 | 0.08 |
| A45 | 5.33 | 0.0000 | 0.00 | 0.04 | 1.01 | 14 | 1.56 | 0.650 | 22 | 1 | 0 | 0 | 2 | 0.14 | 7.3 | 355 | 0.10 |
| A46 | 5.26 | -0.0002 | -0.01 | 0.08 | 1.04 | 6 | 1.53 | 0.655 | 11 | 0 | 0 | 0 | 1 | 0.16 | 9.5 | 802 | 0.08 |
| A47 | 5.26 | -0.0004 | 0.00 | 0.08 | 1.24 | 8 | 1.56 | 0.639 | 0 | 1 | 0 | 0 | 0 | 0.16 | 5.3 | 688 | 0.08 |
| A48 | 5.30 | -0.0009 | 0.01 | 0.08 | 1.05 | 16 | 1.46 | 0.625 | 18 | 0 | 0 | 0 | 2 | 0.15 | 2.6 | 393 | 0.11 |
| A49 | 5.24 | -0.0004 | 0.00 | 0.08 | 1.08 | 10 | 1.56 | 0.638 | 0 | 1 | 0 | 0 | 0 | 0.16 | 5.4 | 729 | 0.08 |
| A50 | 5.26 | -0.0003 | 0.00 | 0.08 | 1.24 | 10 | 1.56 | 0.638 | 0 | 1 | 0 | 0 | 0 | 0.16 | 5.3 | 729 | 0.08 |
| A51 | 5.25 | -0.0004 | 0.00 | 0.08 | 1.09 | 10 | 1.56 | 0.638 | 0 | 1 | 0 | 0 | 0 | 0.16 | 5.4 | 729 | 0.08 |
| A52 | 5.25 | -0.0004 | -0.01 | 0.09 | 1.08 | 10 | 1.53 | 0.638 | 22 | 1 | 1 | 0 | 1 | 0.16 | 11.1 | 742 | 0.08 |
| A53 | 5.27 | -0.0001 | 0.00 | 0.05 | 1.21 | 6 | 1.58 | 0.653 | 0 | 1 | 0 | 0 | 0 | 0.16 | 5.5 | 729 | 0.08 |
| A54 | 5.27 | -0.0010 | 0.00 | 0.06 | 1.08 | 6 | 1.46 | 0.643 | 11 | 0 | 0 | 0 | 2 | 0.16 | 9.2 | 642 | 0.11 |
| A55 | 5.37 | -0.0011 | 0.01 | 0.07 | 0.96 | 6 | 1.42 | 0.644 | 6 | 0 | 0 | 0 | 3 | 0.15 | 9.1 | 679 | 0.08 |
| A56 | 5.33 | -0.0008 | 0.00 | 0.09 | 0.97 | 6 | 1.47 | 0.647 | 34 | 0 | 1 | 0 | 3 | 0.15 | 11.9 | 662 | 0.10 |
| A57 | 5.23 | -0.0006 | 0.01 | 0.07 | 0.90 | 10 | 1.44 | 0.631 | 36 | 0 | 0 | 0 | 3 | 0.16 | 8.5 | 769 | 0.08 |
| A58 | 5.27 | -0.0006 | -0.01 | 0.10 | 1.04 | 8 | 1.52 | 0.660 | 22 | 0 | 0 | 0 | 2 | 0.16 | 12.5 | 807 | 0.08 |
| A59 | 5.29 | -0.0001 | 0.00 | 0.10 | 1.34 | 8 | 1.48 | 0.650 | 13 | 0 | 0 | 0 | 2 | 0.16 | 4.5 | 669 | 0.08 |
| A60 | 5.21 | -0.0008 | -0.01 | 0.06 | 0.95 | 4 | 1.52 | 0.688 | 11 | 1 | 2 | 0 | 2 | 0.15 | 8.1 | 261 | 0.12 |

**[Table 3-3]**

| Compound | Molecular Descriptor | | | | | | | | | | | | | | | | Spectrum Width FWHM [eV] |
|---|---|---|---|---|---|---|---|---|---|---|---|---|---|---|---|---|---|
| | SpDiam_A | AATSC2c | AATSC3s | MATS5s | GATS6c | C3SP2 | AETA_beta | AETA_beta_s | SlogP_VSA5 | n5aRing | n6ARing | nB | nARing | AXp-3d | SaasC | Vabc | |
| A61 | 5.19 | -0.0035 | 0.24 | -0.07 | 1.32 | 6 | 1.40 | 0.650 | 33 | 0 | 1 | 0 | 3 | 0.15 | 4.5 | 218 | 0.15 |
| A62 | 5.19 | -0.0034 | 0.30 | -0.12 | 1.25 | 6 | 1.47 | 0.658 | 27 | 0 | 1 | 0 | 3 | 0.15 | 3.5 | 207 | 0.14 |
| A63 | 5.27 | 0.0001 | -0.02 | -0.06 | 1.12 | 7 | 1.54 | 0.664 | 11 | 1 | 2 | 1 | 2 | 0.15 | 8.7 | 362 | 0.12 |
| A64 | 5.28 | -0.0005 | 0.02 | 0.01 | 0.92 | 6 | 1.59 | 0.656 | 0 | 2 | 0 | 0 | 0 | 0.15 | 1.2 | 311 | 0.13 |
| A65 | 5.24 | -0.0008 | -0.01 | -0.11 | 0.90 | 11 | 1.56 | 0.664 | 22 | 2 | 1 | 0 | 2 | 0.15 | 11.3 | 299 | 0.13 |
| A66 | 5.36 | -0.0004 | 0.00 | 0.07 | 0.80 | 8 | 1.56 | 0.634 | 17 | 0 | 2 | 0 | 2 | 0.15 | 11.4 | 467 | 0.13 |
| A67 | 5.35 | -0.0003 | -0.01 | 0.02 | 1.13 | 6 | 1.53 | 0.660 | 11 | 0 | 3 | 1 | 3 | 0.15 | 14.8 | 468 | 0.14 |
| A68 | 5.43 | 0.0005 | 0.00 | 0.09 | 1.21 | 4 | 1.55 | 0.663 | 0 | 0 | 3 | 1 | 3 | 0.15 | 15.9 | 618 | 0.15 |
| A69 | 5.34 | -0.0003 | 0.00 | 0.05 | 0.87 | 6 | 1.53 | 0.647 | 28 | 0 | 3 | 0 | 3 | 0.15 | 14.7 | 391 | 0.14 |
| A70 | 5.43 | -0.0004 | 0.00 | 0.07 | 0.89 | 6 | 1.51 | 0.650 | 28 | 0 | 3 | 0 | 4 | 0.15 | 18.5 | 578 | 0.12 |
| A71 | 5.23 | -0.0020 | 0.01 | 0.06 | 0.82 | 5 | 1.54 | 0.675 | 6 | 1 | 2 | 0 | 2 | 0.16 | 9.4 | 325 | 0.13 |
| A72 | 5.21 | -0.0010 | -0.01 | 0.04 | 0.78 | 6 | 1.52 | 0.661 | 11 | 1 | 1 | 0 | 2 | 0.15 | 7.9 | 295 | 0.17 |
| A73 | 5.25 | 0.0004 | -0.01 | -0.02 | 0.81 | 4 | 1.52 | 0.674 | 11 | 0 | 1 | 1 | 3 | 0.15 | 12.4 | 395 | 0.14 |
| A74 | 5.17 | -0.0005 | 0.00 | 0.01 | 0.75 | 6 | 1.55 | 0.655 | 11 | 2 | 1 | 0 | 1 | 0.15 | 6.5 | 305 | 0.13 |
| A75 | 5.10 | -0.0019 | -0.06 | -0.04 | 0.85 | 7 | 1.23 | 0.631 | 44 | 0 | 1 | 0 | 3 | 0.16 | 6.0 | 254 | 0.12 |
| A76 | 5.22 | 0.0001 | -0.02 | -0.02 | 0.81 | 6 | 1.33 | 0.654 | 34 | 0 | 1 | 0 | 3 | 0.16 | 10.2 | 441 | 0.13 |
| A77 | 5.32 | -0.0014 | 0.01 | 0.01 | 1.17 | 8 | 1.33 | 0.674 | 27 | 0 | 3 | 0 | 3 | 0.15 | 8.9 | 441 | 0.13 |
| A78 | 5.27 | -0.0005 | 0.02 | 0.06 | 0.92 | 16 | 1.38 | 0.625 | 24 | 4 | 2 | 0 | 2 | 0.14 | 2.9 | 335 | 0.13 |
| A79 | 5.20 | 0.0000 | 0.00 | 0.16 | 0.94 | 16 | 1.44 | 0.611 | 13 | 0 | 0 | 0 | 2 | 0.16 | 0.0 | 399 | 0.16 |
| A80 | 5.43 | -0.0002 | 0.02 | -0.13 | 0.94 | 16 | 1.25 | 0.625 | 37 | 0 | 2 | 0 | 4 | 0.14 | 3.2 | 362 | 0.14 |
| A81 | 5.29 | -0.0001 | 0.01 | 0.00 | 1.07 | 14 | 1.25 | 0.625 | 36 | 0 | 3 | 0 | 5 | 0.15 | 3.1 | 370 | 0.13 |
| A82 | 5.37 | -0.0008 | -0.01 | 0.11 | 1.32 | 10 | 1.51 | 0.670 | 22 | 0 | 0 | 0 | 4 | 0.15 | 16.0 | 557 | 0.12 |

**[Table 4]**

| Compound | Molecular Descriptor | | | | | | | | | | | | | | | | Spectrum Width FWHM [eV] |
|---|---|---|---|---|---|---|---|---|---|---|---|---|---|---|---|---|---|
| | SpDiam_A | AATSC2c | AATSC3s | MATS5s | GATS6c | C3SP2 | AETA_beta | AETA_beta_s | SlogP_VSA5 | n5aRing | n6ARing | nB | nARing | AXp-3d | SaasC | Vabc | |
| B1 | 4.98 | -0.0012 | 0.00 | -0.08 | 1.21 | 4 | 1.58 | 0.650 | 0 | 2 | 0 | 0 | 0 | 0.16 | 0.0 | 150 | 0.72 |
| B2 | 5.16 | -0.0005 | 0.02 | 0.01 | 0.93 | 4 | 1.62 | 0.671 | 0 | 1 | 0 | 0 | 0 | 0.15 | 0.0 | 185 | 0.30 |
| B3 | 4.90 | -0.0009 | 0.02 | -0.26 | 0.48 | 3 | 1.58 | 0.650 | 0 | 2 | 0 | 0 | 0 | 0.15 | 0.0 | 150 | 0.61 |
| B4 | 4.90 | -0.0006 | 0.02 | -0.20 | 0.66 | 2 | 1.62 | 0.683 | 0 | 2 | 0 | 0 | 0 | 0.15 | 0.0 | 144 | 0.72 |
| B5 | 5.15 | -0.0022 | 0.01 | -0.09 | 0.53 | 2 | 1.51 | 0.655 | 11 | 0 | 2 | 0 | 2 | 0.16 | 8.1 | 239 | 0.34 |
| B6 | 5.00 | -0.0038 | 0.03 | -0.39 | 0.15 | 1 | 1.55 | 0.672 | 0 | 1 | 1 | 0 | 1 | 0.16 | 3.0 | 170 | 0.31 |
| B7 | 5.03 | 0.0004 | 0.03 | -0.16 | 0.60 | 3 | 1.62 | 0.671 | 0 | 2 | 0 | 0 | 0 | 0.15 | 0.0 | 185 | 0.53 |
| B8 | 5.07 | -0.0014 | 0.05 | -0.06 | 0.79 | 2 | 1.62 | 0.676 | 0 | 1 | 0 | 0 | 0 | 0.16 | 0.0 | 167 | 0.48 |
| B9 | 5.21 | -0.0003 | 0.00 | 0.02 | 0.54 | 6 | 1.51 | 0.630 | 22 | 0 | 2 | 0 | 2 | 0.16 | 9.8 | 288 | 0.30 |
| B10 | 5.22 | 0.0037 | 0.01 | -0.09 | 1.31 | 8 | 1.54 | 0.647 | 0 | 0 | 2 | 1 | 2 | 0.15 | 7.6 | 336 | 0.31 |
| B11 | 5.13 | 0.0052 | 0.02 | -0.18 | 0.84 | 4 | 1.51 | 0.655 | 0 | 0 | 2 | 1 | 2 | 0.16 | 7.6 | 255 | 0.27 |
| B12 | 5.24 | -0.0004 | 0.01 | 0.02 | 0.58 | 4 | 1.57 | 0.644 | 0 | 0 | 2 | 1 | 2 | 0.15 | 10.7 | 321 | 0.40 |
| B13 | 4.83 | -0.0004 | 0.00 | 0.12 | 1.13 | 4 | 1.57 | 0.571 | 0 | 0 | 0 | 0 | 0 | 0.18 | 0.0 | 162 | 0.27 |
| B14 | 4.99 | -0.0004 | 0.01 | -0.03 | 1.09 | 8 | 1.60 | 0.600 | 0 | 1 | 0 | 0 | 0 | 0.16 | 5.5 | 212 | 0.25 |
| B15 | 5.17 | 0.0002 | 0.08 | -0.12 | 0.54 | 4 | 1.45 | 0.617 | 50 | 0 | 2 | 0 | 2 | 0.16 | 11.7 | 581 | 0.30 |
| B16 | 5.24 | 0.0002 | -0.03 | 0.18 | 1.17 | 10 | 1.29 | 0.571 | 53 | 0 | 0 | 0 | 0 | 0.15 | 2.8 | 350 | 0.25 |

The embodiments disclosed as described above are illustrative in all respects and are not restrictive. The scope of the present invention is indicated by the claims, and includes the meaning equivalent to the claims and all modifications within the scope. In addition, at least some of the embodiments described above may be arbitrarily combined.

The sequences represented in the embodiments described above are not limited, and each processing procedure may be executed by changing the order thereof, or a plurality of pieces of processing may be executed in parallel, unless there is a contradiction. The subject of each processing is not limited, and the processing of each device may be executed by another device, unless there is a contradiction.

### Reference Signs List

- 1: information processing device
- 11: control unit
- 12: storage unit
- 13: communication unit
- 14: display unit
- 15: manipulation unit
- 121: learning model
- 122: compound DB
- 1P: program
- 1A: recording medium
- 2: information terminal device

## Claims

1. A light emitting element, comprising:
an anode;
a cathode; and
a light emitting layer provided between the anode and the cathode,
wherein the light emitting layer contains a light emitting compound satisfying SpDiam_A of 5.0 or more, AATSC2c of 0.003 or less, MATS5s of 0.17 or less, GATS6c of 0.6 or more, and AETA_beta of 1.6 or less in a molecular descriptor by mordred.

2. The light emitting element according to claim 1,
wherein the light emitting compound further satisfies nB of 0 or more and 2 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, n6ARing of 0 or more and 10 or less, nARing of 0 or more and 10 or less, AXp-3d of 0.10 or more and 0.20 or less, SaasC of 0 or more and 40 or less, and Vabc of 100 or more and 2000 or less in the molecular descriptor by mordred.

3. The light emitting element according to claim 1 or 2,
wherein the light emitting layer further contains at least one type selected from the group consisting of a hole transport material, a hole injection material, an electron transport material, an electron injection material, a light emitting material, and an antioxidant.

4. A light emitting compound satisfying SpDiam_A of 5.0 or more, AATSC2c of 0.003 or less, MATS5s of 0.17 or less, GATS6c of 0.6 or more, and AETA_beta of 1.6 or less in a molecular descriptor by mordred.

5. The light emitting compound according to claim 4, further satisfying nB of 0 or more and 2 or less, AATSC3s of -0.2 or more and 0.5 or less, C3SP2 of 0 or more and 30 or less, AETA_beta_s of 0.5 or more and 1.0 or less, SlogPVSA5 of 0 or more and 400 or less, n5aRing of 0 or more and 10 or less, n6ARing of 0 or more and 10 or less, nARing of 0 or more and 10 or less, AXp-3d of 0.10 or more and 0.20 or less, SaasC of 0 or more and 40 or less, and Vabc of 100 or more and 2000 or less in the molecular descriptor by mordred.

6. A composition comprising:
the light emitting compound according to claim 4 or 5; and
at least one type selected from the group consisting of a hole transport material, a hole injection material, an electron transport material, an electron injection material, a light emitting material, an antioxidant, and a solvent.

7. A method for producing a light emitting compound, comprising:
a preparation step of preparing a plurality of compounds; and
an extraction step of extracting a light emitting compound, from the plurality of compounds obtained in the preparation step,
wherein in the extraction step, the light emitting compound satisfying at least one of SpDiam_A of 5.0 or more, AATSC2c of 0.003 or less, MATS5s of 0.17 or less, GATS6c of 0.6 or more, AETA_beta of 1.6 or less, AATSC3s of -0.2 or more and 0.5 or less, C3SP2 of 0 or more and 30 or less, AETA_beta_s of 0.5 or more and 1.0 or less, SlogP_VSA5 of 0 or more and 400 or less, n5aRing of 0 or more and 10 or less, and n6ARing of 0 or more and 10 or less in a molecular descriptor by mordred is extracted.

8. A method for producing a light emitting compound, comprising:
a preparation step of preparing a plurality of compounds; and
an extraction step of extracting a light emitting compound in which a half width of a light emitting spectrum is less than a predetermined value, from the plurality of compounds obtained in the preparation step,
wherein in the extraction step, the light emitting compound satisfying at least one of SpDiam_A of 5.0 or more, AATSC2c of 0.003 or less, MATS5s of 0.17 or less, GATS6c of 0.6 or more, AETA_beta of 1.6 or less, AATSC3s of -0.2 or more and 0.5 or less, C3SP2 of 0 or more and 30 or less, AETA_beta_sof 0.5 or more and 1.0 or less, SlogP_VSA5 of 0 or more and 400 or less, n5aRing of 0 or more and 10 or less, and n6ARing of 0 or more and 10 or less in a molecular descriptor by mordred is extracted.

9. A method for producing a composition, comprising
a step of mixing the light emitting compound produced by the method according to claim 7 or 8 with at least one type selected from the group consisting of a hole transport material, a hole injection material, an electron transport material, an electron injection material, a light emitting material, an antioxidant, and a solvent.

10. A method for producing a light emitting element including an anode, a cathode, and a light emitting layer provided between the anode and the cathode, the method comprising
a step of forming the light emitting layer by using the light emitting compound produced by the method according to claim 7 or 8.

11. An information processing method, comprising:
acquiring a spectral indicator obtained by quantum chemical calculation in a plurality of compounds to be candidates;
classifying,on the basis of the acquired spectral indicator, each of the compounds into a group in which the spectral indicator satisfies a predetermined condition and a group in which the spectral indicator does not satisfy the predetermined condition; and
extracting a light emitting compound classified into the group in which the spectral indicator satisfies the predetermined condition.

12. The information processing method according to claim 11, further comprising:
classifying, on the basis of the spectral indicator and a database storing a known compound, each of the compounds into a group in which the spectral indicator satisfies the predetermined condition and the compound is not stored as the known compound, a group in which the spectral indicator satisfies the predetermined condition and the compound is stored as the known compound, and the group in which the spectral indicator does not satisfy the predetermined condition; and
extracting a light emitting compound classified into the group in which the spectral indicator satisfies the predetermined condition and the compound is not stored as the known compound.

13. The information processing method according to claim 11 or 12, further comprising:
acquiring a value for each molecular descriptor in each of the compounds; and
specifying, on the basis of the acquired value for each of the molecular descriptors in each of the compounds, and the group to which each of the compounds belongs, a combination of the molecular descriptors and a range of the value of each of the molecular descriptors that are capable of separating the group in which the spectral indicator satisfies the predetermined condition from the group in which the spectral indicator does not satisfy the predetermined condition.

14. The information processing method according to any one of claims 11 to 13, further comprising:
acquiring the value for each of the molecular descriptors in each of the compounds; and
specifying, on the basis of the acquired value for each of the molecular descriptors in each of the compounds, and the group to which each of the compounds belongs, a combination of the molecular descriptors and a range of the value of each of the molecular descriptors that are capable of separating the group in which the spectral indicator satisfies the predetermined condition and the compound is not stored as the known compound from the group in which the spectral indicator satisfies the predetermined condition and the compound is stored as the known compound and the group in which the spectral indicator does not satisfy the predetermined condition.

15. The information processing method according to claim 13 or 14, further comprising:
extracting a light emitting compound satisfying the combination of the molecular descriptors and the range of the value of each of the molecular descriptors, which are specified.

16. The information processing method according to any one of claims 13 to 15, further comprising:
specifying, on the basis of mapping data in which a combination value of a plurality of molecular descriptors is mapped for each of the groups, the combination of the molecular descriptors and the range of the value of each of the molecular descriptors.

17. The information processing method according to any one of claims 13 to 16, further comprising:
outputting mapping data in which the combination value of the plurality of molecular descriptors is mapped for each of the groups, and the specified range of the value of each of the molecular descriptors.

18. The information processing method according to any one of claims 13 to 17, further comprising:
outputting the plurality of molecular descriptors and the range of the value of each of the molecular descriptors, which are specified.

19. The information processing method according to any one of claims 13 to 18, further comprising:
extracting the light emitting compound in which the combination of the molecular descriptors and the range of the value of each of the molecular descriptors satisfy SpDiam_A of 5.0 or more, AATSC2c of 0.003 or less, MATS5s of 0.17 or less, GATS6c of 0.6 or more, and AETA_beta of 1.6 or less in a molecular descriptor by mordred.

20. The information processing method according to claim 19,
wherein the light emitting compound in which the combination of the molecular descriptors and the range of the value of each of the molecular descriptors further satisfy nB of 0 or more and 2 or less, AATSC3s of -0.2 or more and 0.5 or less, C3SP2 of 0 or more and 30 or less, AETA_beta_s of 0.5 or more and 1.0 or less, SlogPVSA5 of 0 or more and 400 or less, n5aRing of 0 or more and 10 or less, n6ARing of 0 or more and 10 or less, nARing of 0 or more and 10 or less, AXp-3d of 0.10 or more and 0.20 or less, SaasC of 0 or more and 40 or less, and Vabc of 100 or more and 2000 or less in the molecular descriptor by mordred is extracted.

21. The information processing method according to any one of claims 13 to 20, further comprising:
adding the light emitting compound satisfying the combination of the molecular descriptors and range of the value of each of the molecular descriptors, which are specified, to a database storing the known compound.

22. A method for producing a light emitting compound, comprising:
a step of extracting a light emitting compound by the information processing method according to any one of claims 11 to 21; and
a step of obtaining the extracted light emitting compound.

23. An information processing device, comprising
a control unit executing processing of:
acquiring a spectral indicator obtained by quantum chemical calculation in a plurality of compounds to be candidates;
classifying, on the basis of the acquired spectral indicator, each of the compounds into a group in which the spectral indicator satisfies a predetermined condition and a group in which the spectral indicator does not satisfy the predetermined condition; and
extracting a light emitting compound classified into the group in which the spectral indicator satisfies the predetermined condition.

24. A program for causing a computer to execute processing of:
acquiring a spectral indicator obtained by quantum chemical calculation in a plurality of compounds to be candidates;
classifying, on the basis of the acquired spectral indicator, each of the compounds into a group in which the spectral indicator satisfies a predetermined condition and a group in which the spectral indicator does not satisfy the predetermined condition; and
extracting a light emitting compound classified into the group in which the spectral indicator satisfies the predetermined condition.

25. An information processing method, comprising:
generating a plurality of compounds to be candidates;
acquiring a value for each molecular descriptor in each of the generated compounds;
specifying a spectral indicator by inputting the obtained value of the molecular descriptor of each of the compounds to a model trained to output a spectral indicator of a compound when a value of a molecular descriptor of the compound is input; and
extracting a light emitting compound in which the specified spectral indicator satisfies a predetermined condition.

26. The information processing method according to claim 25,
wherein the model is trained by training data in which the value of the molecular descriptor of the compound is associated with a spectral indicator obtained by quantum chemical calculation.

27. The information processing method according to claim 25 or 26, further comprising:
calculating, for the light emitting compound in which the spectral indicator specified by the model satisfies the predetermined condition, the spectral indicator by the quantum chemical calculation.

28. The information processing method according to any one of claims 25 to 27, further comprising:
storing the spectral indicator and the value of each of the molecular descriptors in association with the light emitting compound in which the spectral indicator satisfies the predetermined condition.

29. The information processing method according to any one of claims 25 to 28, further comprising:
classifying, on the basis of the spectral indicator, each of the compounds into a group in which the spectral indicator satisfies the predetermined condition and a group in which the spectral indicator does not satisfy the predetermined condition.

30. The information processing method according to any one of claims 25 to 29, further comprising:
classifying, on the basis of the spectral indicator and a database storing a known compound, each of the compounds into a group in which the spectral indicator satisfies the predetermined condition and the compound is not stored as the known compound, a group in which the spectral indicator satisfies the predetermined condition and the compound is stored as the known compound, and the group in which the spectral indicator does not satisfy the predetermined condition.

31. The information processing method according to claim 29 or 30, further comprising:
specifying, on the basis of the value of each of the molecular descriptors in each of the compounds, and the group to which each of the compounds belongs, a combination of the molecular descriptors and a range of the value of each of the molecular descriptors that are capable of separating the group in which the spectral indicator satisfies the predetermined condition or the group in which the spectral indicator satisfies the predetermined condition and the compound is not stored as the known compound from the other group.

32. The information processing method according to claim 31, further comprising:
specifying, on the basis of mapping data in which a combination value of a plurality of molecular descriptors is mapped for each of the groups, the combination of the molecular descriptors and the range of the value of each of the molecular descriptors.

33. The information processing method according to claim 31 or 32,
wherein a molecular descriptor with a high contribution ratio in the model is preferentially used to specify the combination of the molecular descriptors.

34. A method for producing a light emitting compound, comprising:
a step of extracting a light emitting compound by the information processing method according to any one of claims 25 to 33; and
a step of obtaining the extracted light emitting compound.

35. A method for providing a light emitting compound, comprising:
outputting a light emitting compound extracted from a plurality of compounds to be candidates generated by a computer, wherein the light emitting compound in which a spectral indicator specified using a model trained to output a spectral indicator of a compound when a value of a molecular descriptor of the compound is input, satisfies the predetermined condition.

36. A data creation method, comprising:
storing a plurality of compounds to be candidates generated by a computer, and a spectral indicator calculated by quantum chemical calculation with respect to each of the compounds in association with each other; and
further storing a value for each molecular descriptor in each of the compounds in association with each of the compounds.

37. The data creation method according to claim 36,
wherein the spectral indicator of each of the compounds is specified by inputting the value of the molecular descriptor of each of the compounds to a model trained to output a spectral indicator of a compound when a molecular descriptor of the compound is input,
the method further comprising:
storing the compound in which the spectral indicator specified by the model satisfies a predetermined condition.

38. The data creation method according to claim 36 or 37, further comprising:
storing a flag according to the spectral indicator calculated by the quantum chemical calculation with respect to each of the compounds i in association with each of the compounds.

39. The data creation method according to any one of claims 36 to 38, further comprising:
storing a flag indicating whether the compound is a known compound in association with each of the compounds.

40. The data creation method according to any one of claims 36 to 39, further comprising:
specifying, on the basis of the flag stored in association with each of the compounds, the compound in which the spectral indicator satisfies the predetermined condition or the compound that is not the known compound.

41. The data creation method according to any one of claims 36 to 40, further comprising:
newly creating, when the known compound is obtained, for the known compound, a flag indicating that the compound is the known compound.

42. An information processing method, comprising:
acquiring a condition of a desired property for a compound;
generating a plurality of compounds to be candidates by a computer;
acquiring a value for each molecular descriptor of each of the generated compounds;
extracting the compound in which the property of the compound satisfies the condition from each of the generated compounds by using a model trained to output a property of a compound when a value of a molecular descriptor of the compound is input;
calculating a property by quantum chemical calculation with respect to the extracted compound;
specifying the compound in which the property calculated by the quantum chemical calculation satisfies the condition; and
outputting the specified compound and the property of the compound.

43. The information processing method according to claim 42, further comprising:
classifying, on the basis of the property of each of the compounds, and a database storing a known compound, each of the compounds into a group in which the property of the compound satisfies the condition and the compound is not stored as the known compound, a group in which the property of the compound satisfies the condition and the compound is stored as the known compound, and a group in which the property of the compound does not satisfy the condition.

44. The information processing method according to claim 42 or 43, further comprising:
specifying, on the basis of the value of each of the molecular descriptors of each of the compounds, a combination of the molecular descriptors and a range of the value of each of the molecular descriptors that are capable of separating the group in which the property of the compound satisfies the condition and the compound is not stored as the known compound from the other group; and
outputting a plurality of molecular descriptors and the range of the value of each of the molecular descriptors, which are specified.

45. An information processing method, comprising:
receiving a condition of a desired property for a compound;
acquiring a plurality of molecular descriptors of the compound and a range of a value of each of the molecular descriptors satisfying the received condition; and
displaying the plurality of molecular descriptors and the range of the value of each of the molecular descriptors, which are acquired.

46. The information processing method according to claim 45, further comprising:
displaying information indicating a contribution ratio of the molecular descriptor in extraction processing of the compound satisfying the condition.
